# EUROPÄISCHE PATENTSCHRIFT

(11) **EP 3 058 516 B1**
(45) Veröffentlichungstag und Bekanntmachung des Hinweises auf die Patenterteilung: **01.08.2018**
(21) Anmeldenummer: 14790019.5
(22) Anmeldetag: 07.10.2014
(51) Int. Cl.: G06M 1/04, A61M 15/00

(54) **ZÄHLWERK UND HANDGERÄT MIT ZÄHLWERK**
COUNTER AND HANDHELD DEVICE WITH COUNTER
COMPTEUR ET APPAREIL PORTATIF ÉQUIPÉ D'UN COMPTEUR

(30) Priorität: 15.10.2013 DE 102013111381; 06.10.2014 DE 102014114462
(43) Veröffentlichungstag der Anmeldung: 24.08.2016
(73) Patentinhaber: Von Schuckmann, Alfred, 47627 Kevelaer (DE)
(72) Erfinder: Von Schuckmann, Alfred, 47627 Kevelaer (DE)
(74) Vertreter: Müller, Enno
(86) Internationale Anmeldenummer: PCT/EP2014/071427
(87) Internationale Veröffentlichungsnummer: WO 2015/055463

(56) Entgegenhaltungen:
- DE-A1- 10 061 723
- DE-A1-102005 033 398
- FR-A1- 2 858 867

## Beschreibung

Die Erfindung betrifft zunächst ein Zählwerk nach den Merkmalen des Oberbegriffes des Anspruches 1.

Weiter betrifft die Erfindung ein Handgerät mit einem Zählwerk nach den Merkmalen des Oberbegriffes des Anspruches 14. Derartige Zählwerke sind bereits in verschiedener Hinsicht bekannt geworden. Es wird beispielsweise auf die WO 2006/051073 A1 (US 7448342 B2, US 7827984 B2) verwiesen. Weiter auch etwa auf die WO 2007/045904 A1.

Bei einem aus der US 6283365 B1 bekannten Zählwerk ist das Antriebsteil als Teil der feststehenden Drehachse ausgebildet. In Einzelheit ist das Antriebsteil auch zweiteilig ausgebildet, wobei das Zählrad bei einem Gesamthub sowohl auf einem Hin- wie einem Rückweg mit unterschiedlichen Bereichen des Antriebsteils zusammenwirkt. Das Beaufschlagungsteil verlagert unmittelbar das durch ein Fenster in dem Beaufschlagungsteil sichtbare Zählrad, welches weiter in einer Reibanlage zu dem Beaufschlagungsteil drehbewegbar ist.

Bei einem aus der WO 2007/124406 A2 bekannten Zählwerk ist das Antriebsteil über Schrägflächen bewegbar, die zur Zusammenwirkung mit entsprechenden Schrägflächen an dem Beaufschlagungsteil ausgebildet sind. Das Beaufschlagungsteil vollzieht nur eine hin- und her gehende Bewegung in Richtung der Drehachse. Eine erforderliche Drehbewegung des Antriebsteils wird nur durch die Zusammenwirkung zwischen den Schrägflächen des Antriebsteils und des Beaufschlagungsteils erreicht.

Bei einem aus der DE 10061723 A1 bekannten Zählwerk bzw. Handgerät mit einem Zählwerk ist ein Schaltelement vorgesehen, das durch ein hierzu nur axial bewegliches Beaufschlagungsteil, eine Betätigungstaste bzw. eine Spraydüse, zur Zählung beaufschlagt wird. Die Drehung erfolgt durch eine außenseitige Führung um eine lediglich geometrische Drehachse.

Bei dem eingangs genannten Zählwerk bzw. Handgerät mit Zählwerk wie es aus der WO2006/051073 A1 bekannt ist, wird das Zählrad durch ein Planetenrad gedreht, das seinerseits auf einem unterseitig verzahnten Sonnenrad aufsitzt. Das Sonnenrad ist durch einen Schrittschaltfinger beaufschlagt. Soweit das Sonnenrad als Antriebsteil zu sehen ist, dreht es sich lediglich um eine geometrische Drehachse.

Ausgehend von dem zuletzt genannten Stand der Technik beschäftigt sich die Erfindung mit der Aufgabe, ein gut funktionierendes, einfach ausgebildetes Zählwerk bzw. ein Handgerät mit einem solchen Zählwerk anzugeben.

Diese Aufgabe ist zunächst beim Gegenstand des Anspruches 1 gelöst, wobei darauf abgestellt ist, dass das Beaufschlagungsteil drehbewegt ist und mit dem Antriebsteil zur gemeinsamen Drehung verbunden ist und dass das Antriebsteil einen Führungsabschnitt aufweist, mit dem es im zusammengesetzten Zustand des Zählwerks auf der als feststehender Zapfen ausgebildeten ersten Drehachse des Zählwerks aufsitzt.

Diese Aufgabe ist auch beim Gegenstand des Anspruches 27 gelöst, wobei darauf abgestellt ist, dass das Zählwerk ein Bodenteil aufweist, das einen Teil des Gehäuses bildet und in dieses eingesetzt ist, wobei sich das Bodenteil in Richtung der Längsachse des Substanzbehältnisses zumindest teilweise in Überdeckung zu diesem befindet und dass das Bodenteil eine feststehende erste Drehachse für das Zählrad trägt, auf welcher bevorzugt auch das Antriebsteil geführt ist, wobei sich das Antriebsteil bezüglich des Zählrades auf der Seite des Substanzbehältnisses befindet.

Das Antriebsteil und das Beaufschlagungsteil können als einheitliches Teil ausgebildet sein. Sie können auch als zueinander bewegliche, jedoch in Drehrichtung gekoppelte Teile ausgebildet sein. Bei einer einteiligen Ausbildung können sie auch insbesondere materialeinheitlich integral geformt sein, beispielsweise als einheitliches Kunststoffspritzteil.

Das Beaufschlagungsteil dient bevorzugt zur Übertragung einer Bewegung eines Substanzbehälters, in dem die pharmazeutische Substanz aufgenommen ist, insbesondere bei einer Ausgabebetätigung des Substanzbehälters, auf das Antriebsteil. Wesentlich ist, dass sich eine Drehbewegung des Antriebsteils ergibt. Eine darüber hinaus gegebene axiale Bewegung des Antriebsteils kann gegeben sein, sie kann aber auch entfallen. Wenn die Drehbewegung durch Einwirkung auf das Beaufschlagungsteil erzeugt wird, wird sie auch bei einer teilemäßigen Trennung von Beaufschlagungsteil und Antriebsteil unmittelbar auf das Antriebsteil übertragen.

Die Drehbewegung des Beaufschlagungsteils bzw. des Antriebsteils ist bevorzugt eine hin- und hergehende Drehbewegung. Die hin- und hergehende Bewegung wird, soweit nicht ohnehin eine Einteiligkeit vorliegt, jeweils von dem Antriebsteil mit vollzogen. Bevorzugt dreht sich das Beaufschlagungsteil und/oder das Antriebsteil innerhalb des Handgerätes immer um einen gleichen vorgegebenen Winkelbetrag, ausgehend von einer praktisch immer gleichen Ausgangsstellung.

Zufolge der Drehkopplung zwischen dem Beaufschlagungsteil und dem Antriebsteil, insbesondere bei einer fest verbundenen oder integralen Ausbildung des Beaufschlagungsteils und des Antriebsteils, ergibt sich entsprechend bei einer Betätigung auch eine Drehung des Beaufschlagungsteils relativ zu dem Substanzbehälter. Insofern kann prinzipiell vorgesehen sein, dass der Bereich der zur gemeinsamen Drehung verbundenen Teile, des Beaufschlagungs- und des Antriebsteils, der mit dem Substanzbehälter zusammenwirkt, drehbar aber in Richtung der Drehachse fest verbunden mit dem Bereich ausgebildet ist, der das Antriebsteil bildet oder auf dieses einwirkt. Hierbei kommt es nicht notwendig zu einer Drehung zwischen dem Substanzbehälter und dem Beaufschlagungsteil. Es kann aber auch bei entsprechender Drehung des Beaufschlagungsteils, insbesondere wenn ein solcher eigens drehbarer Bereich nicht vorgesehen ist, eine unmittelbare Drehreibung zwischen dem Substanzbehälter und dem Beaufschlagungsteil bei einer Ausgabebetätigung des Substanzbehälters entstehen.

Das Beaufschlagungsteil ist bevorzugt überlagert zu einer Bewegung in Richtung der Drehachse drehbewegt. Zugleich mit einer Bewegung in Richtung der Drehachse vollzieht sich also in diesem Fall die Drehbewegung des Beaufschlagungsteils, wenn entsprechend auf den Substanzbehälter eingewirkt wird.

Das Antriebsteil und/oder das Beaufschlagungsteil weisen weiter bevorzugt eine Eingriffsausformung auf, die im Zuge einer entsprechenden Verlagerung des Beaufschlagungsteils bzw. des Antriebsteils mit einer Eingriffsöffnung und/oder einer Eingriffsfläche des Zählrades, welche Eingriffsfläche ggf. als Begrenzung der Eingriffsöffnung ausgebildet sein kann, in Eingriff steht oder in Eingriff kommt. Entsprechend sind die Teile Antriebsteil - ggf. als entsprechender Bereich des Beaufschlagungsteils - und das Zählrad ständig in Eingriff miteinander oder nicht ständig in Eingriff miteinander.

Die Drehachse, zu der das Antriebsteil und damit auch das Beaufschlagungsteil drehbar ist, ist bevorzugt im Hinblick auf ein Gehäuse, in welchem das Zählwerk eingesetzt ist, feststehend.

Das Antriebsteil kann nicht nur eine Eingriffsausformung zur Zusammenwirkung mit dem Zählrad aufweisen, sondern auch eine Getriebeausformung. Mit der Getriebeausformung kann gesondert auf das Antriebsteil bzw. das kombinierte Beaufschlagungs-und Antriebsteil zu dessen Drehung einwirkbar sein. Die Zusammenwirkung zwischen dem Antriebsteil und dem Zählrad, soweit sie zu einer Drehung des Zählrades führt, erfolgt bei einer in Richtung der Drehachse gegebenen Relativbewegung zwischen dem Antriebsteil und dem Zählrad.

Soweit vor- und nachstehend auf das Antriebsteil Bezug genommen ist, ist hiermit der entsprechende Bereich des kombinierten Beaufschlagungs- und Antriebsteils angesprochen. Bei der bevorzugten einteiligen Ausführung entsprechend auch das kombinierte Teil insgesamt.

So ist es bevorzugt, dass das Antriebsteil koaxial zu der Drehachse angeordnet ist.

Das Antriebsteil kann vor einer Verlagerung vollständig von dem Zählrad getrennt sein, so dass es das Zählrad nicht berührt. Gleichwohl kann in einer rechtwinkelig zu der Drehachse gegebenen Horizontalebene noch eine Überdeckung zwischen dem Antriebsteil und dem Zählrad gegeben sein. Bevorzugt ist eine solche Überdeckung nur zwischen einer nach unten vorstehenden Eingriffsausformung des Antriebsteils und einer Begrenzung der Eingriffsöffnung, in der Regel der genannten Eingriffsfläche, gegeben. Es kann auch vorgesehen sein, dass vor einer Verlagerung keine Horizontalebene gegeben ist, in welcher zwischen dem Antriebsteil und dem Zählrad noch eine Überdeckung gegeben ist.

Bevorzugt ist auch, dass das Antriebsteil vor einer Verlagerung an der ersten Drehachse gefangen ist. Diese Verbindung kann beispielsweise durch eine zwischen dem Antriebsteil und der Drehachse wirkende Feder gegeben sein. Die Feder kann eine Rückstellfeder sein, die das Antriebsteil in die Stellung vor der Verlagerung beaufschlagt.

Die erste Drehachse kann zapfenartig, mit einem freien oberen Ende, ausgebildet sein. Die Drehachse kann einen Vorsprung, bspw. als umlaufende Schulter ausgebildet, aufweisen, auf welchem die Rückstellfeder aufsitzen kann.

Die Getriebeausformung des Antriebsteils und/oder des Beaufschlagungsteils kann aus einer Kulisse bestehen. Sie kann auch aus einem Kulissenstein bestehen. Sie kann auch aus einem Verzahnungsabschnitt bestehen.

Ein mit der Getriebeausformung zusammenwirkendes Getriebeteil kann außerhalb des eigentlichen Zählwerkes, beispielsweise in einem Gehäuse des Handgerätes, in welches das Zählwerk einzusetzen ist, ausgebildet sein.

Alternativ kann das Getriebeteil auch an einem Bereich des Zählwerkes, insbesondere der feststehenden Drehachse, ausgebildet sein. Das Getriebeteil kann in jeweiliger Anpassung an die verwirklichte Getriebeausformung ausgebildet sein. So kann das Getriebeteil bei einer Ausbildung der Getriebeausformung als Kulisse aus einem Kulissenstein bestehen. Bei einer Ausformung der Getriebeausformung als Kulissenstein entsprechend aus einer Kulisse. Bei einer Ausformung der Getriebeausformung als Verzahnungsabschnitt besteht entsprechend das Getriebeteil aus einem zum Verzahnungseingriff mit dem Verzahnungsabschnitt ausgebildeten weiteren Verzahnungsabschnitt.

Bevorzugt ist weiter, dass das Zählwerk einen Teil des Gehäuses des Handgerätes umfasst. Das Handgerät, in welches das Zählwerk einzusetzen ist, weist hierbei entsprechend hinsichtlich seines Gehäuses einen Fehlbereich auf. Dieser ist erst ergänzt und damit das Gehäuse vervollständigt, wenn das Zählwerk in das Handgerät eingesetzt ist.

Eine oder mehrere Federn, wobei beispielsweise zwei Federn vorgesehen sein können, welche auf das Antriebsteil einwirken, können sich auch, bevorzugt alternativ, gegebenenfalls aber auch kombinativ zu einer Abstützung auf der Drehachse, auf einer Innenseite des genannten Teils des Gehäuses, welches bei dieser Ausführungsform zu dem Zählwerk gehört, abstützen. Eine feste Verbindung zwischen der Drehachse und dem Gehäuse kann insbesondere auch eine materialeinheitliche integrale Verbindung sein. Die feste Verbindung ist bevorzugt jedenfalls derart ausgebildet, dass zwischen dem genannten Teil des Gehäuses und der Drehachse keine Bewegung möglich ist.

Eine oder zwei, oder gegebenenfalls auch mehr Federn können sich seitlich zu einem Zählrad und in Richtung der ersten Drehachse das Zählrad übergreifend erstrecken. Wenn zwei mit dem Antriebsteil zusammenwirkende Federn vorgesehen sind, können diese bezüglich der ersten Drehachse sich gegenüberliegend zueinander erstrecken.

Hinsichtlich der Federeinwirkung kann auch vorgesehen sein, dass eine Feder unmittelbar zwischen dem Beaufschlagungsteil und der Drehachse wirkt und eine weitere Feder zwischen dem Antriebsteil und dem Beaufschlagungsteil. Letztere Feder ist dann insbesondere dazu vorgesehen, eine Bewegung in Richtung der Drehachse, die auch unabhängig von einer solchen Feder bevorzugt vorgesehen ist, zwischen dem Antriebsteil und dem Beaufschlagungsteil zu ermöglichen.

Das Antriebsteil kann an dem Beaufschlagungsteil in Richtung der Drehachse beweglich, jedoch in Drehrichtung praktisch unbeweglich gefangen sein. Insbesondere kann es hiermit rastverbunden sein.

Bei einer bevorzugten Ausführungsform erfolgt die Drehung des Antriebsteils auch allein aus der genannten Zusammenwirkung zwischen der Getriebeausformung und dem Getriebeteil.

Weiter ist bevorzugt, dass mehrere Zählräder vorgesehen sind. Diese mehreren Zählräder können insbesondere koaxial zueinander angeordnet sein. Darüber hinaus können die Zählräder koaxial zu der Drehachse und/oder in Richtung der Drehachse übereinander angeordnet sein. Die Zusammenwirkung einer Eingriffsausformung mit einer Eingriffsöffnung ist hier bevorzugt nur bzgl. eines Zählrades, weiter bevorzugt nur bzgl. des dem Antriebsteil unmittelbar zugeordneten Zählrades, (das in der Regel das oberste Zählrad ist,) gegeben.

Das Antriebsteil kann als Eingriffsausformung einen Eingriffszapfen aufweisen. Der Eingriffszapfen kann achsparallel zu der Drehachse verlaufen.

Ein Zählrad, jedenfalls bevorzugt das unmittelbar dem Antriebsteil zugeordnete Zählrad, kann eine oder mehrere Eingriffsöffnungen aufweisen. Eine Eingriffsöffnung kann zu einer Aufnahme des Eingriffszapfens ausgebildet und angeordnet sein.

Eine Eingriffsöffnung ist bevorzugt mit einer Eingriffsfläche ausgebildet. Die Eingriffsfläche kann gekrümmt oder schräg bezüglich einer Vertikalen verlaufen, so dass sich in Bezug auf eine Bewegung der Eingriffsausformung, bspw. des Eingriffszapfens, wenn diese vorgesehen ist, in Richtung der Drehachse, eine Bewegung des Zählrades in Umfangsrichtung zu der Drehachse, also eine Drehbewegung, ergibt. Diese Drehbewegung durch die entsprechende Gestaltung der Eingriffsfläche ist bevorzugt überlagert zu der Drehbewegung, die sich aufgrund der Zusammenwirkung des Antriebsteils mit dem Gehäuse zufolge der Getriebeausformung ergibt.

Zwischen zwei in Richtung der Drehachse unmittelbar benachbarten Zählrädern kann ein Übertragungszahnrad angeordnet sein. Das Übertragungszahnrad kann um eine zweite Drehachse drehbar sein. Weiter kann das Übertragungszahnrad sich quer zu seiner zweiten Drehachse in Überdeckung zu beiden Zählrädern erstrecken. Durch eine nur in einem bestimmten Umfangsbereich des oberen Zählrades ausgebildete Verzahnung wird das untere Zählrad mit Hilfe des Übertragungszahnrades nur gedreht, wenn zufolge zuvor erfolgter Drehung des unteren Zählrades dieser bestimmte, mit Verzahnung ausgebildete Bereich in Eingriff kommt mit dem Übertragungszahnrad.

Das Übertragungszahnrad kann drehbar an einem Ausleger der ersten Drehachse gehaltert sein. Der Ausleger kann die zweite Drehachse ausbilden.

Insbesondere können zwei Übertragungszahnräder vorgesehen sein. Diesbezüglich ist es auch bevorzugt, zwei Ausleger vorzusehen. In diesem Fall können auch weiter beide Ausleger in Richtung der ersten Drehachse beabstandet sein.

Eines, mehrere oder alle Zählräder können mit einer Rückdrehsperre zusammenwirken. Eine Rückdrehsperre kann als ein auf eine die Zeichen aufweisenden Außen-Umfangsfläche eines Zählrades einwirkendes Sperrteil ausgebildet sein. Ein Sperrteil kann als federbares Hebelteil ausgebildet sein. Es kann einen frei ausfederbaren und bevorzugt mit dem jeweiligen Zählrad in Eingriff stehenden freien Endbereich aufweisen und einen Halterungs-Endbereich, der bevorzugt integral einheitlich mit einer Halterung des Hebelteils an dem Gehäuse ausgebildet ist.

Ein bevorzugt fingerartig gebildetes Sperrteil kann sich quer zu einer Erstreckung der ersten Drehachse erstrecken.

Das Sperrteil kann an dem bereits genannten Teil des Gehäuses, welches in diesem Fall Teil des Zählwerks ist, befestigt sein. Es kann aufgrund nur elastischer Verformung wirken.

Gegenstand der Erfindung ist auch ein Handgerät zur Ausgabe pharmazeutischer Substanzen, insbesondere eines Inhaliermedikamentes, mit einem Gehäuse, wobei in dem Gehäuse ein mit einem Ausgabehub zu betätigendes Substanzbehältnis, das eine Längsachse aufweist, und ein Zählwerk aufgenommen ist, wobei das Zählwerk mindestens ein, lesbare Zeichen aufweisendes, Zählrad und ein zum Drehen des Zählrades auf dieses einwirkendes, bei einem Ausgabehub von dem Substanzbehältnis verlagertes Antriebsteil aufweist.

Auch insofern wird auf den eingangs genannten Stand der Technik verwiesen.

Zur vorteilhaften Ausbildung eines Handgerätes, insbesondere eines solchen Handgerätes wie vorstehend merkmalsmäßig angegeben, ist vorgesehen, dass das Zählwerk ein Bodenteil aufweist, das einen Teil des Gehäuses bilden kann und in dieses einsetzbar sein kann, wobei sich das Bodenteil in Richtung der Längsachse des Substanzbehältnisses zumindest teilweise in Überdeckung zu diesem befindet und eine feststehende erste Drehachse für das Zählrad trägt, auf welcher auch das Antriebsteil angeordnet ist, wobei sich das Antriebsteil gesehen von dem Bodenteil bezüglich des Zählrades auf der Seite des Substanzbehältnisses befindet.

Das Bodenteil kann integraler aber nicht materialeinheitlicher Bestandteil des Gehäuses sein, derart, dass es einen Teil der Außenfläche des Gehäuses im eingesetzten Zustand bildet. Bevorzugt in einem Bodenbereich des Gehäuses. Ein Bodenbereich des Gehäuses ist der in Richtung des Substanzbehältnisses untere Bereich, der auch die Aufnahme für einen Ausgabefortsatz des Substanzbehältnisses aufweist.

Eine weitere Lösung bei einem wie vorausgesetzt gegebenen Handgerät ist auch dadurch gegeben, dass das Antriebsteil und/oder ein auf das Antriebsteil einwirkendes Beaufschlagungsteil eine Getriebeausformung aufweist zur Zusammenwirkung mit einem gehäusefesten Getriebeteil, zur Erzeugung einer Drehbewegung des Antriebsteils relativ zu dem Gehäuse im Zuge einer Verlagerung.

Des Weiteren kann das Handgerät hinsichtlich des Zählwerkes eines oder mehrere der weiteren in Bezug auf das Zählwerk bereits erläuterten Merkmale aufweisen.

Ein derartiges Handgerät ist insbesondere zur Ausgabe sprühfähiger Substanzen ausgebildet. Auf die in den eingangs genannten Druckschriften erwähnten Substanzen wird beispielhaft verwiesen.

Nachstehend ist die Erfindung des Weiteren anhand der beigefügten Zeichnung erläutert, die jedoch lediglich ein Ausführungsbeispiel wiedergibt. Hierbei zeigt:
- Fig. 1: ein Handgerät erster Ausführungsform zur Ausgabe sprühfähiger Substanzen, in einer Ansicht von schräg vorne;
- Fig. 2: das Handgerät gemäß Figur 1 in einer Ansicht von schräg hinten;
- Fig. 3: eine erste Explosionsdarstellung des Handgeräts, Substanzbehältnis und Zählwerk erster Ausführungsform;
- Fig. 4: eine zweite Explosionsdarstellung des Zählwerks erster Ausführungsform alleine, in einer Ansicht von schräg oben;
- Fig. 5: eine Darstellung gemäß Figur 4, in einer Ansicht von schräg unten;
- Fig. 6: eine Querschnittsdarstellung des Handgerätes, geschnitten entlang der Linie VI - VI in Figur 2;
- Fig. 7: eine Herausvergrößerung des Bereiches VII - VII in Figur 6;
- Fig. 8: eine Rückansicht des Handgerätes erster bzw. zweiter Ausführungsform;
- Fig. 9: einen Querschnitt durch den Gegenstand gemäß Figur 8 erster Ausführungsform, geschnitten entlang der Linie IX - IX;
- Fig. 10: einen Querschnitt durch den Gegenstand gemäß Figur 7, geschnitten entlang der Linie X - X;
- Fig. 11: einen Querschnitt durch den Gegenstand gemäß Figur 7, geschnitten entlang der Linie XI - XI;
- Fig. 12: einen Querschnitt durch den Gegenstand gemäß Figur 7, geschnitten entlang der Linie XII - XII;
- Fig. 13: einen Querschnitt durch den Gegenstand gemäß Figur 7, geschnitten entlang der Linie XIII - XIII;
- Fig. 14: eine schematische aufgeschnittene Seitenansicht des Handgerätes erster Ausführungsform mit darin angeordnetem Zählwerk;
- Fig. 15: eine Ansicht gemäß Figur 14, in perspektivischer Darstellung, gesehen von schräg oben;
- Fig. 16: eine weitere Darstellung gemäß Figur 1, mit der Verdeutlichung einer weiteren Schnittebene;
- Fig. 17: einen Schnitt durch den Gegenstand gemäß Figur 16, geschnitten entlang der Linie XVII - XVII;
- Fig. 18: einen Querschnitt durch den Gegenstand gemäß Figur 16 bzw. Figur 17, geschnitten in der Ebene XVIII - XVIII in Figur 17;
- Fig. 19: eine Darstellung gemäß Figur 17, bei niedergedrücktem Substanzbehältnis,
- Fig. 20: einen Querschnitt durch den Gegenstand gemäß Figur 19, geschnitten in der Ebene XX - XX;
- Fig. 21: eine Darstellung gemäß Figur 3 einer weiteren Ausführungsform;
- Fig. 22: eine Darstellung gemäß Figur 4 der weiteren Ausführungsform;
- Fig. 23: eine Darstellung gemäß Figur 5 der weiteren Ausführungsform;
- Fig. 24: eine Querschnittsdarstellung der weiteren Ausführungsform geschnitten entlang der Linie XXIV - XXIV in Figur 2;
- Fig. 25: eine Herausvergrößerung des Bereichs XXV - XXV in Figur 24;
- Fig. 26: einen Querschnitt durch den Gegenstand gemäß Figur 8, bei Ausbildung der weiteren Ausführungsform, geschnitten entlang der Linie XXVI - XXVI;
- Fig. 27: einen Querschnitt durch den Gegenstand gemäß Figur 25, geschnitten entlang der Linie XXVII - XXVII;
- Fig. 28: einen Querschnitt durch den Gegenstand gemäß Figur 25, geschnitten entlang der Linie XXVIII - XXVIII;
- Fig. 29: einen Querschnitt durch den Gegenstand gemäß Figur 25, geschnitten in der Ebene XXIX - XXIX,
- Fig. 30: einen Querschnitt durch den Gegenstand gemäß Figur 25, geschnitten entlang der Linie XXX - XXX;
- Fig. 31: eine schematisch aufgeschnittene Seitenansicht des Handgerätes mit darin angeordnetem Zählwerk der weiteren Ausführungsform;
- Fig. 32: eine Ansicht gemäß Figur 31 in perspektivischer Darstellung, gesehen von schräg oben;

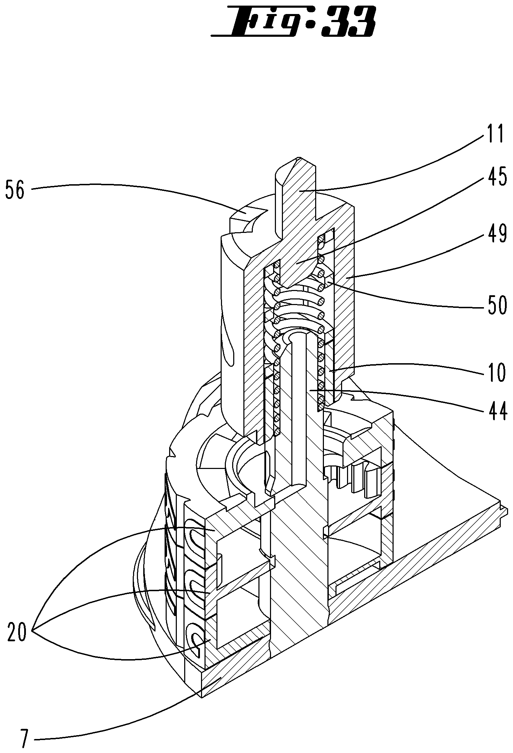

- Fig. 33: eine herausvergrößerte Schnittdarstellung des Zählwerks allein, geschnitten entlang einer Durchmesserlinie der Drehachse;
- Fig. 34: eine Darstellung gemäß Figur 33, bei einem anderen Drehwinkel der Schnittebene; und
- Fig. 35: eine Seitenansicht auf das mit dem Beaufschlagungsteil verbundene Antriebsteil.

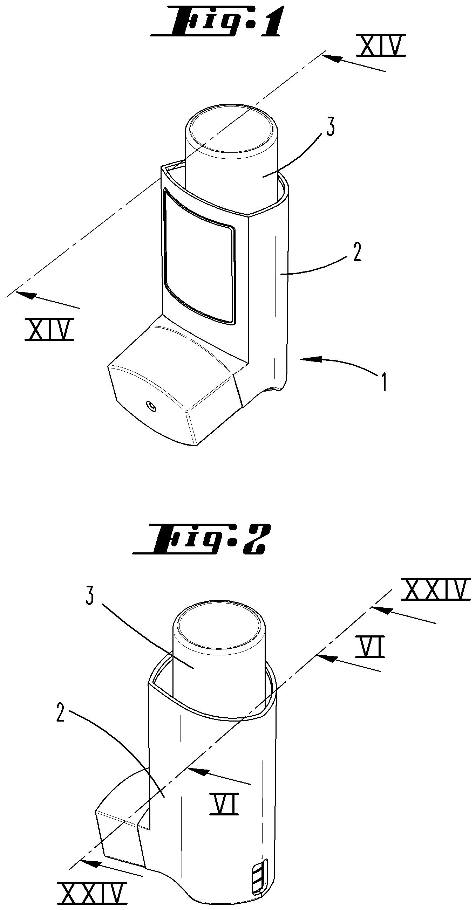

Dargestellt und beschrieben ist, zunächst mit Bezug zu den Figuren 1 bis 3, ein Handgerät 1 zur Ausgabe sprühfähiger Substanzen, insbesondere Inhaliermedikamenten.

Das Handgerät 1, das auch als Inhaler bezeichnet wird, weist ein Gehäuse 2 auf und ein darin entnehmbar aufgenommenes Substanzbehältnis 3. Das Substanzbehältnis 3 wird auch als Kartusche oder Kanister bezeichnet.

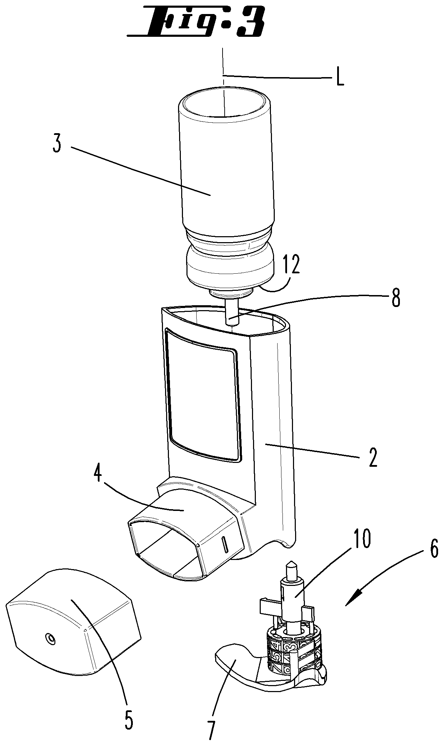

Bei dem in den Figuren 1 und 2 dargestellten Handgerät 1 ist ein von dem Benutzer gewöhnlich in den Mund zu nehmender Ausgabestutzen 4 (Figur 3), auch als Mundstück bezeichnet, von einer Verschlusskappe 5 überfangen.

In dem Handgerät 1 ist ein Zählwerk 6 aufgenommen. Es handelt sich bei den Ausführungsbeispielen bevorzugt um ein Einzeldosis-Zählwerk. Entsprechend erfolgt eine Zählung bzw. eine Weiterdrehung des Einer-Zählrades bei jeder Betätigung des Gerätes, also bspw. bei jeder Auf-und-ab-Bewegung des Medikamentenbehälters. Das Zählwerk 6 weist bevorzugt ein Bodenteil 7 auf. Weiter bevorzugt, wie bei den Ausführungsbeispielen auch vorgesehen, bildet das Bodenteil 7 einen Teil des Gehäuses 2, einschließlich eines entsprechenden Teils der Außenfläche des Gehäuses. Das Bodenteil 7 befindet sich seitlich zu dem Mundstück, d.h. dem Ausgabestutzen 4, in dem unteren an das Mundstück seitlich anschließenden Teil des Gehäuses 2.

Die Ausgestaltung des Zählwerks kombiniert mit einem Bodenteil, das zugleich einen ergänzenden Teil des Gehäuses bildet, ist auch insofern vorteilhaft, als das Zählwerk - mit dem Bodenteil - in das Handgerät eingesetzt werden kann, wenn sich das Substanzbehältnis bereits darin befindet. Es kann in entgegengesetzter Richtung zu dem Substanzbehältnis in das Gehäuse 2 eingesetzt werden.

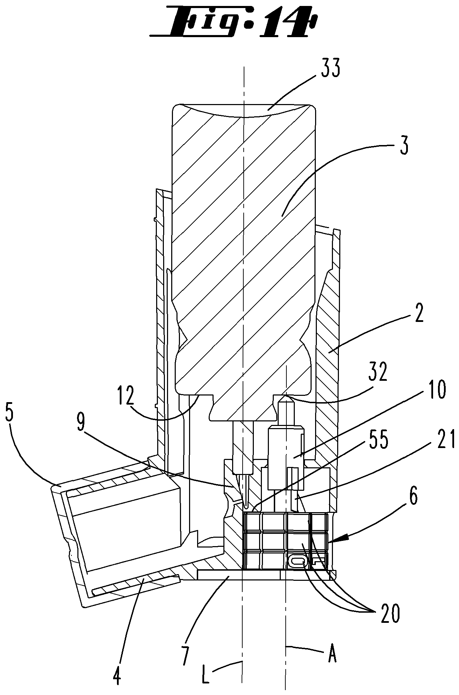

Zur Ausgabe von Substanz ist bei den Ausführungsbeispielen das Substanzbehältnis 3 in Richtung seiner Längsachse, bevorzugt von Hand, zu betätigen. Das Substanzbehältnis weist hierzu im Einzelnen einen dornartigen Ausgabefortsatz 8 auf, der, für solche Substanzbehältnisse 3 in bekannter Weise, gegen Federkraft zur Ausgabe in Richtung auf das Innere des Substanzbehältnis 3 zu bewegen ist. Über eine in dem Gehäuse 2 ausgebildete Aufnahme 9 (vgl. insbesondere Figur 14) für den Ausgabefortsatz 8 wird bei einem solchen Ausgabehub austretende Substanz umgelenkt und tritt im Sinne eines Sprühens in das Innere des Ausgabestutzens 4 ein.

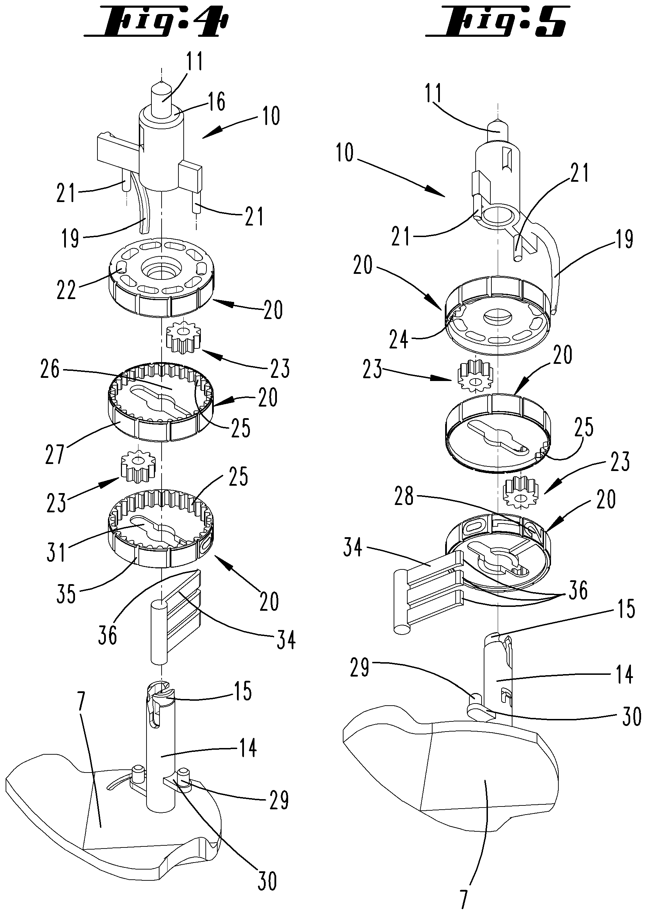

Mit Bezug zu den Figuren 4 bis 15 ist das Zählwerk 6 und dessen Anordnung in dem Handgerät 1 in weiterer Einzelheit erläutert.

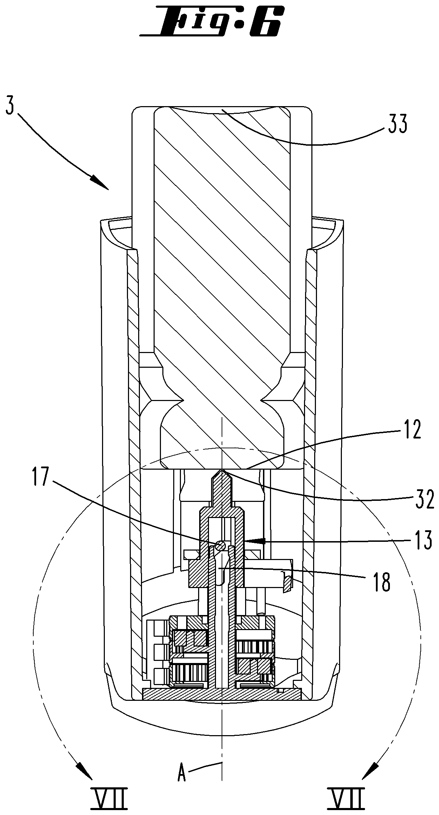

Das Zählwerk 6 weist, als zugleich oberstes Teil, da hier bei diesem Ausführungsbeispiel kombiniert mit dem Beaufschlagungsteil ausgebildet, ein Antriebsteil 10 auf. Das Antriebsteil 10 wird bei einem Ausgabehub durch das Substanzbehältnis 3 beaufschlagt. Es ist zugleich auch ein Beaufschlagungsteil. Bei der bevorzugten einteiligen Ausführung des Antriebsteils 10 mit dem Beaufschlagungsteil dreht sich das Beaufschlagungsteil entsprechend auch auf der zugeordneten Fläche des Substanzbehältnisses im Zuge einer Betätigung. Das Antriebsteil 10 weist im Hinblick auf die Ausbildung auch als Beaufschlagungsteil einen Beaufschlagungsfortsatz 11 auf, der bei entsprechender Betätigung mit einer nach unten weisenden Stirnfläche 12 des Substanzbehältnis 3 zusammenwirkt, vergleiche etwa Figur 6.

Das Antriebsteil 10 weist weiter bevorzugt einen, beim Ausführungsbeispiel und bevorzugt mittleren, Führungsabschnitt 13 (Figur 6) auf, mit dem es im zusammengesetzten Zustand des Zählwerks 6 auf der als feststehender Zapfen ausgebildeten ersten Drehachse 14 des Zählwerks aufsitzt bzw. diese umgibt. Bevorzugt sitzt es oberseitig und kappenartig abschließend bezüglich eines oberen Stirnendes 15 der ersten Drehachse 14 auf dieser auf.

Der Führungsabschnitt 13 ist entsprechend bevorzugt im Wesentlichen als Hohlzylinder gebildet. Oberseitig weist er eine Stirnfläche 16 auf, von welcher sich erhebend der Beaufschlagungsfortsatz 11 ausgebildet ist.

Bevorzugt innenseitig ist an dem Führungsabschnitt 13 als Getriebeausformung ein Kulissenstein 17 ausgebildet, der mit einer Kulisse 18 als Getriebeteil an der ersten Drehachse 12 zusammenwirkt. Die Kulisse 18 erstreckt sich im Wesentlichen in Richtung einer geometrischen Achse A der ersten Drehachse 14. Der Kulissenstein 17 ist bevorzugt in weiterer Einzelheit ein einen Hohlraum des Führungsabschnitts 13 querender Zapfen bzw. eine in beiden gegenüberliegenden Wandbereichen des Führungsabschnitts 13 verankerter Stangenabschnitt.

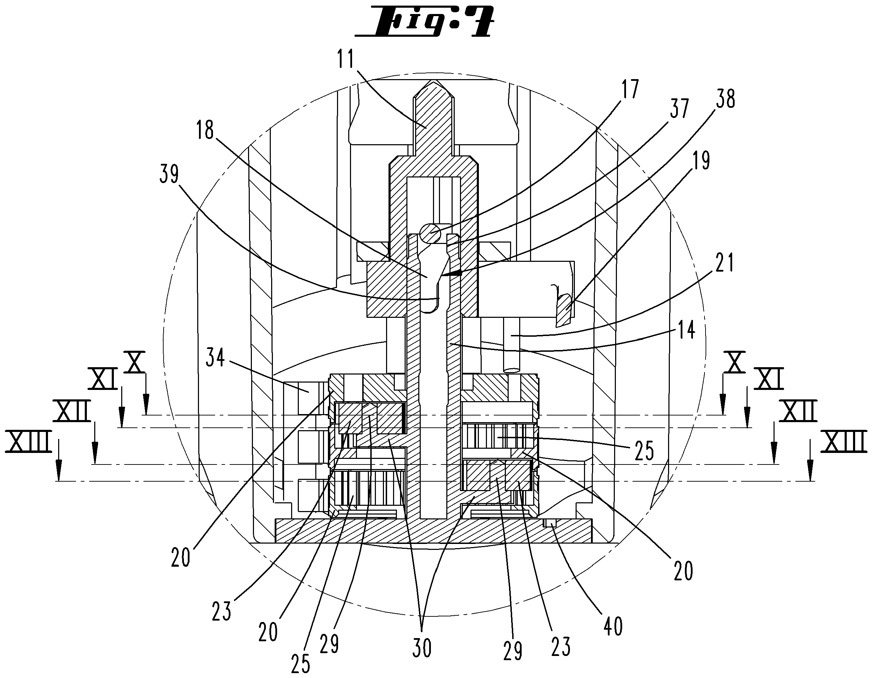

Die Kulisse 18 ist im Einzelnen und bevorzugt in drei Abschnitte unterteilt. Einen ersten Einführabschnitt 37. Der Einführabschnitt 37 erbringt bzw. ermöglicht eine ungedrehte Bewegung in Richtung auf den Fuß der Drehachse 14 des Antriebsteils 10 zu Beginn eines Niederdrückens des Substanzbehältnisses 3. Es folgt ein Drehabschnitt 38. Hierbei sind bevorzugt zwei gegenüberliegende, in der Querschnittsdarstellung der Figur 7 als Schrägabschnitte erscheinende Kulissenflächen 39 ausgebildet, die bezogen auf eine Draufsicht auf die erste Drehachse 14 gegensinnig verlaufen.

Wie im Übrigen ersichtlich und bevorzugt, ist die Drehachse 14 auch als Hohlzylinder ausgebildet.

An den zweiten Abschnitt 37 schließt sich in Richtung auf einen Boden der Drehachse 14 schließlich ein weiterer Abschnitt 39 der Kulisse an. Der Abschnitt 39 ermöglicht wiederum eine ungedrehte Niederbewegung des Antriebsteils 10 bei weiterem Niederdrücken des Substanzbehältnisses 3.

Das Antriebsteil 10 ist über eine Feder 19 in einer unbetätigten Stellung des Zählwerks bzw. des Handgerätes an dem Bodenteil 7 abgestützt. Die so gegebene Ausgangsstellung des Antriebsteils 10 ist eine bezüglich der ersten Drehachse 14 oberste Stellung des Antriebsteils 10.

In dieser Ausgangsstellung, d. h. der Stellung des Antriebsteils 10 vor einer Verlagerung zum Antrieb eines Zählrades 20 ist das Antriebsteil 10 vollständig von einem solchen (ersten) Zählrad 20 getrennt.

Das Antriebsteil 10 weist weiter als Eingriffsausformung einen bzw. beim Ausführungsbeispiel zwei Eingriffszapfen 21 auf. Die Eingriffszapfen 21 dienen zum Drehantrieb eines Zählrades 20.

Bevorzugt ist das Antriebsteil 10 mit der Feder 19 und/oder den Eingriffszapfen 21 und/oder dem Beaufschlagungsfortsatz 11 und/oder dem Führungsabschnitt 13 materialeinheitlich einteilig, bspw. als Kunststoffspritzteil, ausgebildet.

Beim Ausführungsbeispiel und bevorzugt weist das Zählwerk 6 drei untereinander angeordnete Zählräder 20 auf. Das oberste bzw. dasjenige Zählrad, das unmittelbar dem Antriebsteil 10 zugeordnet ist, weist über seinen Umfang verteilt eine Reihe von Eingriffsöffnungen 22 auf. Eine Eingriffsausformung bzw. ein Eingriffszapfen 21 kann in eine Eingriffsöffnung 22 einfahren.

Die Eingriffsöffnung 22 ist ersichtlich und bevorzugt kreisabschnittsförmig gebildet. Mit einem Radius ausgehend von der geometrischen Achse A der ersten Drehachse 14. Da beim Ausfahren der Eingriffszapfen 21, die mit einer gewissen Rückdrehung des Antriebsteils 10 relativ zu dem Zählrad 20 einhergehen, entsprechend eine Rückbewegung der Eingriffszapfen 21 noch in den Eingriffsöffnungen 22 eintritt, ist die genannte kreisabschnittsförmige Gestaltung vorteilhaft. In Umfangsrichtung sind, auf einer Kreislinie mit demselben Radius, bevorzugt mehrere Eingriffsöffnungen 22 hintereinander ausgebildet. Bei der Ausführungsform sind es beispielsweise zehn Eingriffsöffnungen. Bei einer Betätigung sind bevorzugt zwei Eingriffsausformungen des Antriebsteils in zwei gegenüberliegenden Eingriffsöffnungen 22 jeweils eingefahren.

Die Eingriffsöffnung weist eine Eingriffsfläche auf, gegen welche die Eingriffsausformung zur Anlage kommen kann.

In Längsrichtung der geometrischen Achse A der ersten Drehachse 14 sind zwischen zwei Zählrädern 20 und diese übergreifend Übertragungszahnräder 23 angeordnet.

Ein Übertragungszahnrad 23 wirkt einerseits mit ersten Zähnen 24 eines bezüglich des Übertragungszahnrades 23 oberen Zählrades 20 (bezogen auf einen üblichen Gebrauchszustand des Handgerätes) und andererseits mit zweiten Zähnen 25 eines unteren Zählrades 20 zusammen. Ersichtlich sind die ersten Zähne 24 nur über einen Teil, weiter speziell einen geringen Teil des Umfangs des diesbezüglichen Zählrades 20 ausgebildet, während die zweiten Zähne 25 über den gesamten Umfang des betreffenden Zählrades 20 ausgebildet sind.

Ein Zählrad 20, das wie hier als mittleres Zählrad zu drei übereinander angeordneten Zählrädern 20 angeordnet ist, kann, gegebenenfalls gesondert durch einen Boden 26, sowohl erste Zähne 24 sowie auch zweite Zähne 25 aufweisen.

Das in einer solchen Übereinander-Anordnung unterste Zählrad 20 braucht nur zweite Zähne 25 aufzuweisen.

Die ersten bzw. zweiten Zähne 24, 25 sind bevorzugt auf einer Innenfläche eines Umfangs-Mantelteils 27 des betreffenden Zählrades ausgebildet. Die Umfangs-Mantelfläche 27 ist weiter bevorzugt mit der gewünschten Anzahl von einzelnen lesbaren Zeichen 28 ausgebildet. Ein Zeichen 28 kann hierbei aufgedruckt sein oder eingeprägt sein oder spitztechnisch mit ausgeformt sein oder in sonstiger Weise erzeugt sein.

Ein Übertragungszahnrad 23 ist auf einer zweiten Drehachse 29 geführt, die bevorzugt für sich ebenfalls feststehend ist.

Weiter bevorzugt, wie beim Ausführungsbeispiel ersichtlich, ist eine zweite Drehachse 29 auf einem Ausleger 30 der ersten Drehachse 14 angeordnet. Der Ausleger 30 erstreckt sich radial zu der geometrischen Achse A der ersten Drehachse 14.

Ein Boden 6 eines Zählrades 20 kann eine schlüssellochartige Öffnung 31 aufweisen, welche sowohl ermöglicht, dass die Drehachse 14 das Zählrad 20 durchsetzt wie auch dass das Zählrad über einen oder, beim Ausführungsbeispiel, zwei Ausleger 30 übergreifend auf die Drehachse 14 aufgesetzt wird.

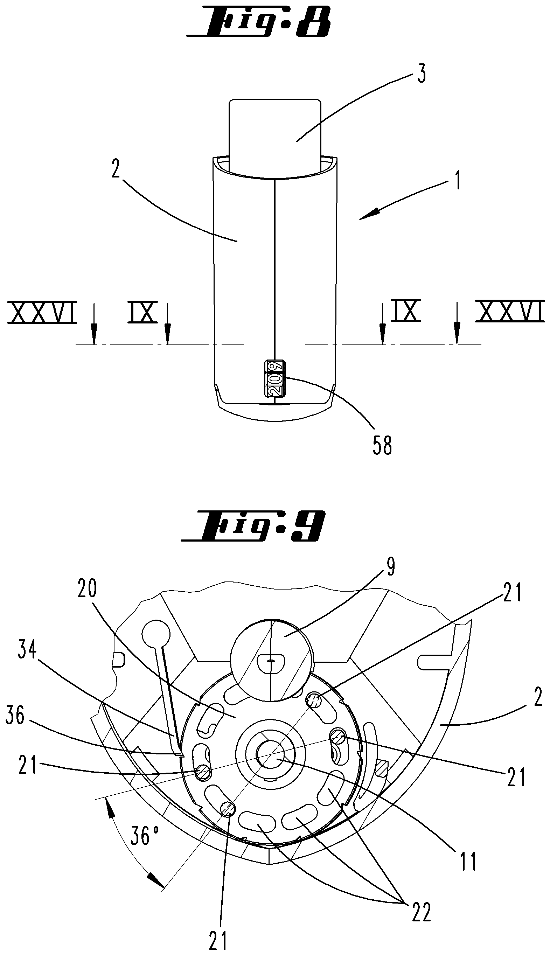

Im Einzelnen ist das Zusammenwirken der Zählräder 20 und der Übertragungszahnräder 23 unter Bezug auf die Figuren 9 bis 13 ergänzend erläutert, die entsprechende Querschnitte durch das Zählwerk, wie in Figur 7 dargestellt, wiedergeben.

Der Querschnitt der Figur 9, siehe Figur 8, zeigt eine Draufsicht auf das oberste Zählrad 20, mit eingetauchtem Eingriffszapfen 21. Es sind die beiden Stellungen der Eingriffszapfen vor Beginn einer Verdrehung des Zählrades 20 und nach Abschluss der Verdrehung des Zählrades 20 aber vor Herausfahren der Eingriffszapfen 21 dargestellt. Beim Ausführungsbeispiel ist die erfolgte Winkelbewegung ersichtlich mit 36 Grad angegeben. Die eingetauchten Eingriffszapfen liegen bei diesem Ausführungsbeispiel jeweils an einer, an einer Vertikalen ausgerichteten Eingriffsfläche während der Verdrehung des Zählrades 20 an.

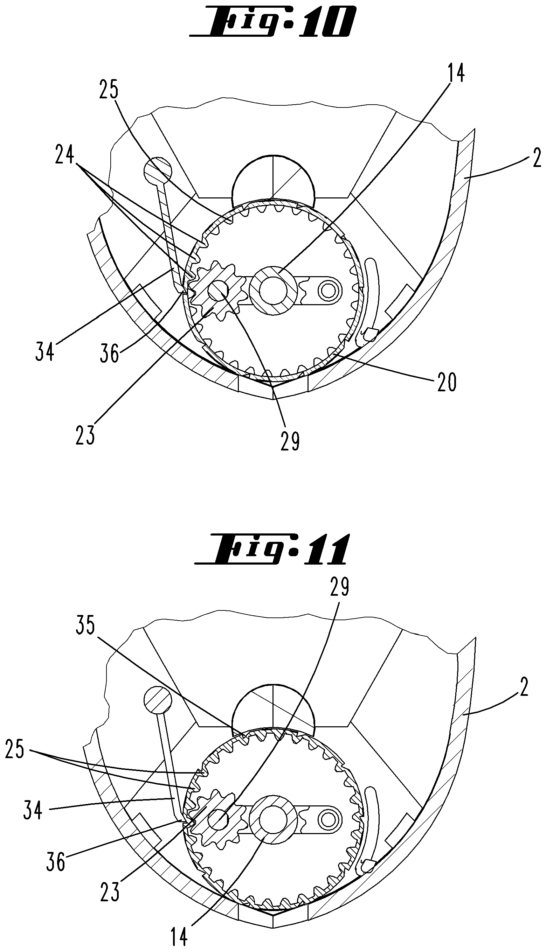

Aus der Querschnittsdarstellung der Figur 10 ist ersichtlich, dass die über den Umfang nur teilweise ausgebildeten ersten Zähne 24 mit dem ersten (obersten) Übertragungszahnrad 23 zusammenwirken können (bei entsprechender Umfangsstellung des obersten Zählrades 20).

Die Querschnittsdarstellung der Figur 11 zeigt dann, dass dasselbe Übertragungszahnrad 23 mit den zweiten Zähnen 25 des auf das erste Zählrad 20 folgenden darunter befindlichen weiteren Zählrades 20 in ständigem Eingriff ist.

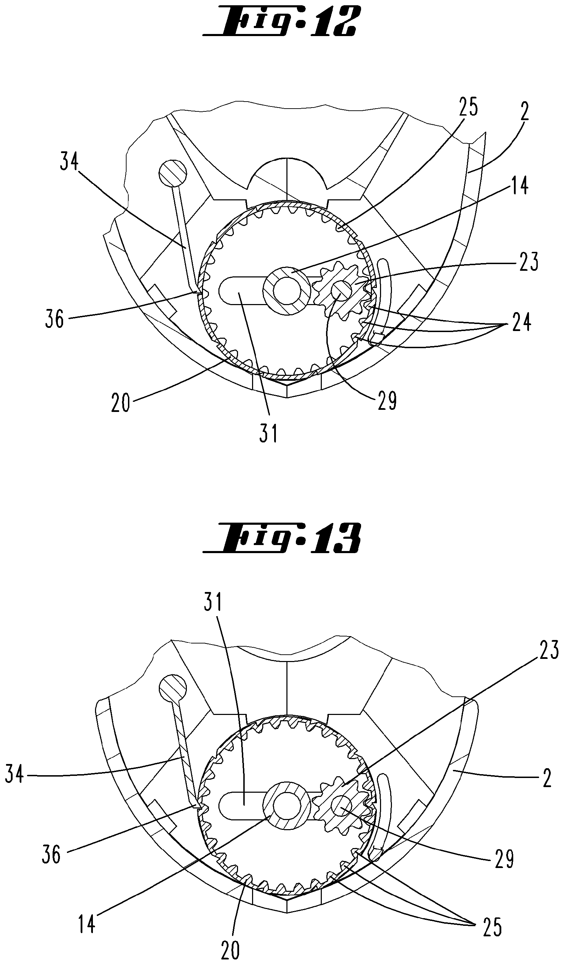

Gleiche Verhältnisse ergeben sich bezüglich der Zusammenwirkung zwischen diesem (von oben gesehen) zweiten Zählrad und dem darunter befindlichen dritten Zählrad 20, vgl. Figuren 12 und 13.

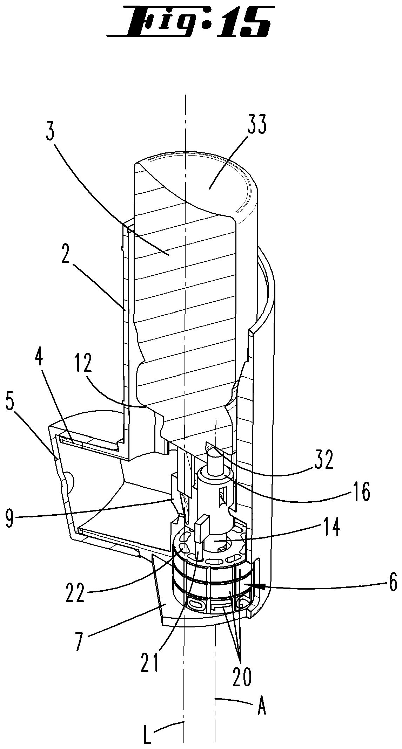

Mit Bezug zu den Figuren 14 und 15 ist auch zu erkennen, dass die geometrische Achse A zwar in einer Seitenansicht gemäß Figur 14 seitlich versetzt zu der Längsachse L des Substanzbehältnisses 3 verläuft. Gleichwohl aber das Bodenteil bzw. Gehäuseteil 7 in vertikaler Überdeckung, zumindest zu einem wesentlichen Grundrissbereich des Substanzbehältnisses 3 angeordnet ist.

Das Antriebsteil 10 wirkt gleichsam (als Gegenkraft) exzentrisch auf das Substanzbehältnis 3 bezogen auf die Längsachse L ein. Hierbei wirkt das Antriebsteil 10 auch nicht mit einer aus Sicht des Ausgabefortsatzes 8 des Substanzbehältnisses 3 ersten Stirnfläche, sondern mit einer zweiten, gegenüber der ersten Stirnfläche stufenmäßig abgesetzten und einen größeren Radiusbereich aufweisenden zweiten Stirnfläche zusammen. Dies erbringt eine größere Bauraumnutzung für das Zählwerk 6.

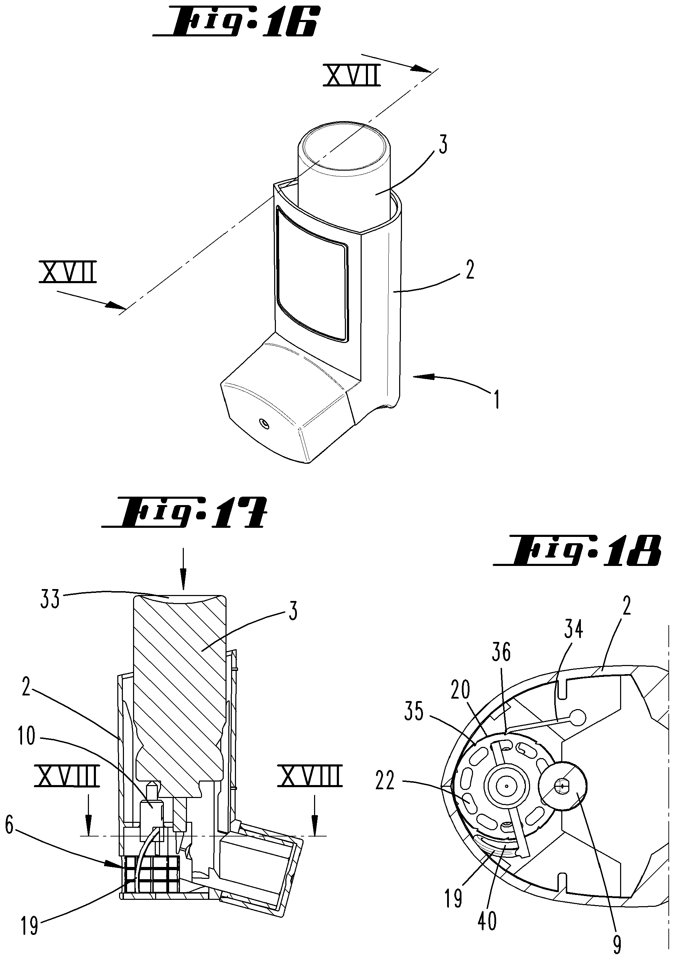

Unter Bezug auf die Figuren 16 bis 20 ist ergänzend erläutert und dargestellt die Wirkung und Bewegung der Feder 19, die auf das Antriebsteil 10 einwirkt. In den Figuren 17 und 18 befindet sich das Antriebsteil 10 in seiner Ausgangsstellung. Eine Einwirkung auf das Substanzbehältnis hat in diesem Zustand noch nicht stattgefunden.

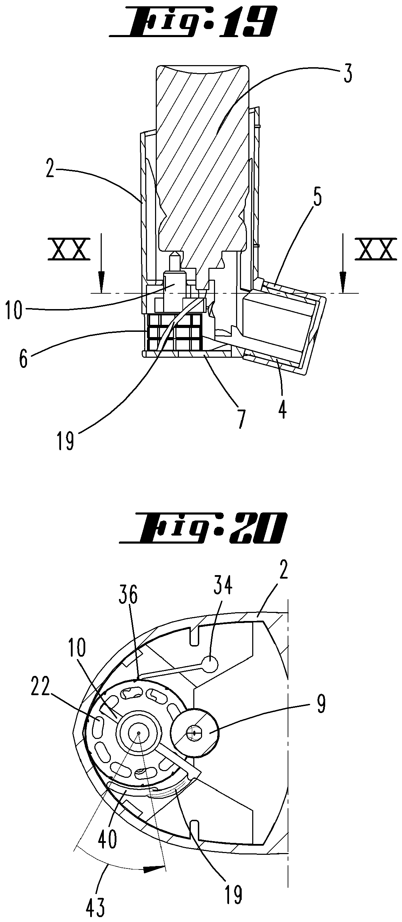

In Figur 19 ist das Substanzbehältnis 3 niedergedrückt (ohne dass ein betätigender Finger eines Benutzers dargestellt ist). Ersichtlich ist die Feder 19 stärker gekrümmt und, wie sich aus Figur 20 ergibt, insgesamt in Bezug auf Nut 40, in der ein Fußbereich der Feder aufgenommen ist, in dieser Nut 40 Drehbewegungsrichtung des Antriebsteils 10 bei dieser Betätigung auch, vorbewegt.

Bei einer Benutzung des Handgeräts bzw. einer Betätigung des Zählwerkes ergibt sich folgendes:

Wie aus den Figuren 6 und 7 ersichtlich, ist in einer Ausgangsstellung das Antriebsteil 10 mit einer Berührungsspitze 32 des Beaufschlagungsfortsatzes 11 in Anlage an der Stirnfläche 12 des Substanzbehältnisses 3.

Bei einer Betätigung des Handgerätes wird, beispielsweise mit Fingerdruck, auf die obere Stirnseite 33 des Substanzbehältnisses 3 gedrückt, so dass dieses in Figur 6 nach unten bewegt wird.

Damit wird das Antriebsteil 10 relativ zu der feststehenden Drehachse 14 nach unten bewegt. Der Kulissenstein 17 bewirkt hierbei durch Zusammenwirkung mit der Kulisse 18 eine Verdrehung des Antriebsteils 10 relativ zu, d. h. drehend um die Drehachse 14.

Zugleich fahren - beim Ausführungsbeispiel zwei - Eingriffszapfen 21 in die Eingriffsöffnungen 22 des diesbezüglich obersten Zählrades 20 ein. Sie bewegen sich in den Eingriffsöffnungen 22 bis zum Anschlag an eine Eingriffsfläche und nehmen dann das entsprechende Zählrad 20 um einen Winkelbetrag von beispielsweise 36 Grad oder 40 Grad in Umfangsrichtung mit. Falls das Übertragungszahnrad 23 hierbei mit den ersten Zähnen 24 in Kontakt kommt, wird zugleich auch das darunter befindliche bzw. das mit dem Übertragungszahnrad 23 gekoppelte weitere Zählrad 20 bewegt. In besonderen Fällen dann auch zugleich das weiter darunter befindliche, durch das weitere Übertragungszahnrad 23 angetriebene weitere Zählrad 20.

Wenn der Benutzer den Druck auf die Stirnfläche 33 aufhebt, bewegt sich das Substanzbehältnis 3 zufolge einer eigenen, in dem Ausgabeteil enthaltenen Feder, in seine Ausgangsstellung zurück.

Das Antriebsteil 10 vollzieht diese Rückbewegung zufolge der auf sie wirkenden Feder 19 mit, so dass es sich danach wieder in dem Zustand gemäß Figur 6 befindet.

Bei einer Drehung eines Zählrades 20 erfolgt auch ein Überlaufen einer zugeordneten Rückdrehsperre 34. Hierfür sind bevorzugt an dem Zählrad sich in Richtung der geometrischen Achse A der Drehachse 14 erstreckende Rastkerben 35 ausgebildet, in welche eine Rückdrehsperre 34 mit einer vorderen Sperrnase 36 dann wieder eingreift. Ein Rückdrehen des Zählrades 20 ist so verhindert.

Die genannten Teile des Zählwerkes können bevorzugt einzeln oder sämtlich jeweils Kunststoffspritzteile sein. Bspw. aus PP oder PE.

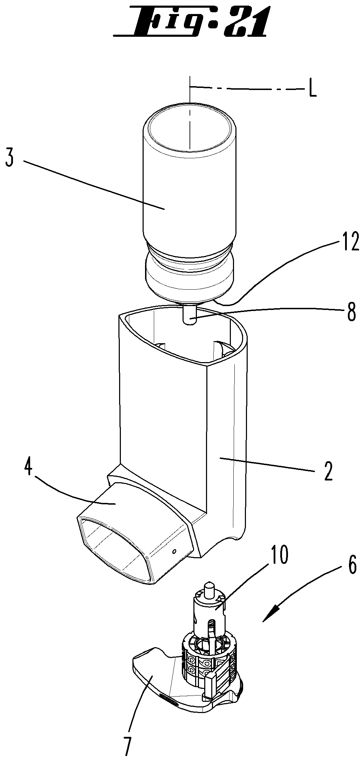

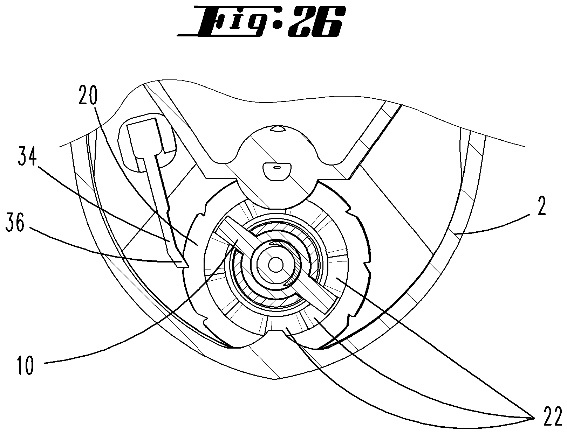

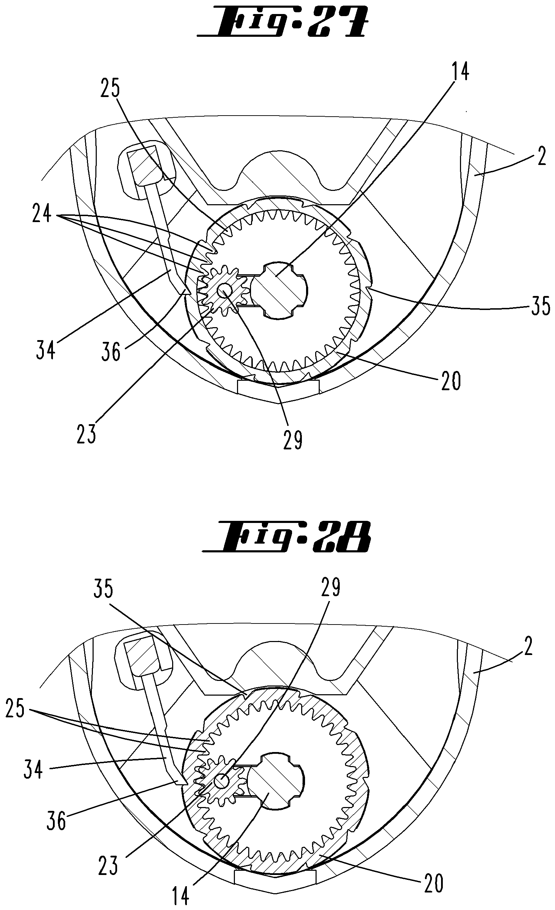

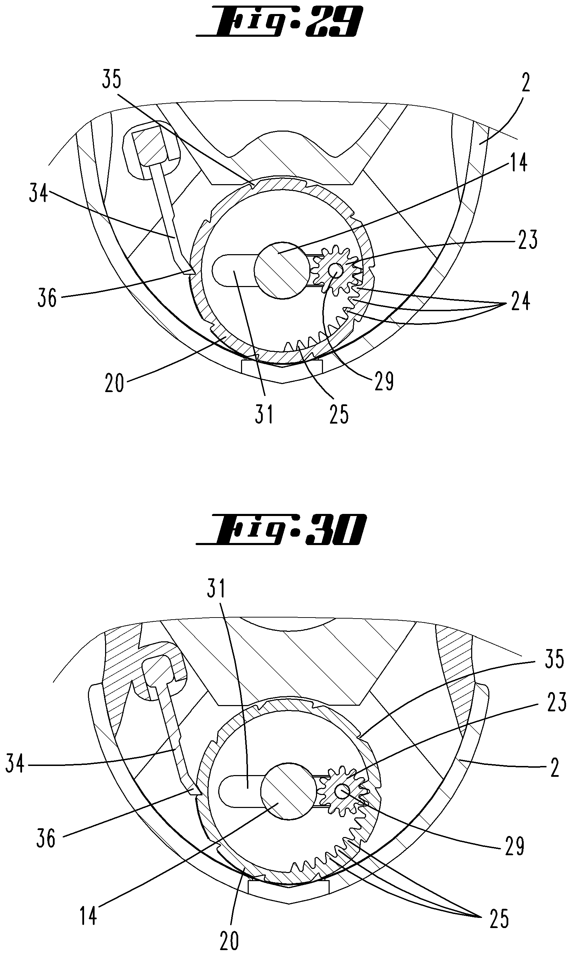

Mit Bezug zu den Figuren 21 bis 32 ist eine weitere Ausführungsform des Zählwerks bzw. des Handgerätes beschrieben.

Soweit Übereinstimmung besteht, ist die Beschreibung hinsichtlich einiger Teile, welche selbe Bezugszeichen aufweisen, nicht in jeder Hinsicht wiederholt. Die vorherige Beschreibung hat entsprechend auch bezüglich solcher Teile für diese zweite Ausführungsform Gültigkeit. Umgekehrt sind in Bezug auf diese zweite Ausführungsform auch Erläuterungen zu Teilen mit selben Bezugszeichen in der ersten Ausführungsform gegeben, die dort nicht oder nicht in jeder Einzelheit so beschrieben sind. Diese Beschreibung zu der zweiten Ausführungsform hat entsprechend dann auch Gültigkeit zu der ersten Ausführungsform, soweit nicht gerade eine spezifische Abänderung bezüglich der zweiten Ausführungsform vorliegt.

Wie aus Figur 21 zu entnehmen ist, ist auch bei dieser Ausführungsform vorgesehen, dass das Zählwerk mit einem Bodenteil, hier in Form einer Bodenplatte 7, verbunden ist, die auch zugleich, auf ihrer dem Zählwerk abgewandten Seite, eine Außenseite des so zusammengesetzten Gehäuses 2 des Handgerätes 1 bildet.

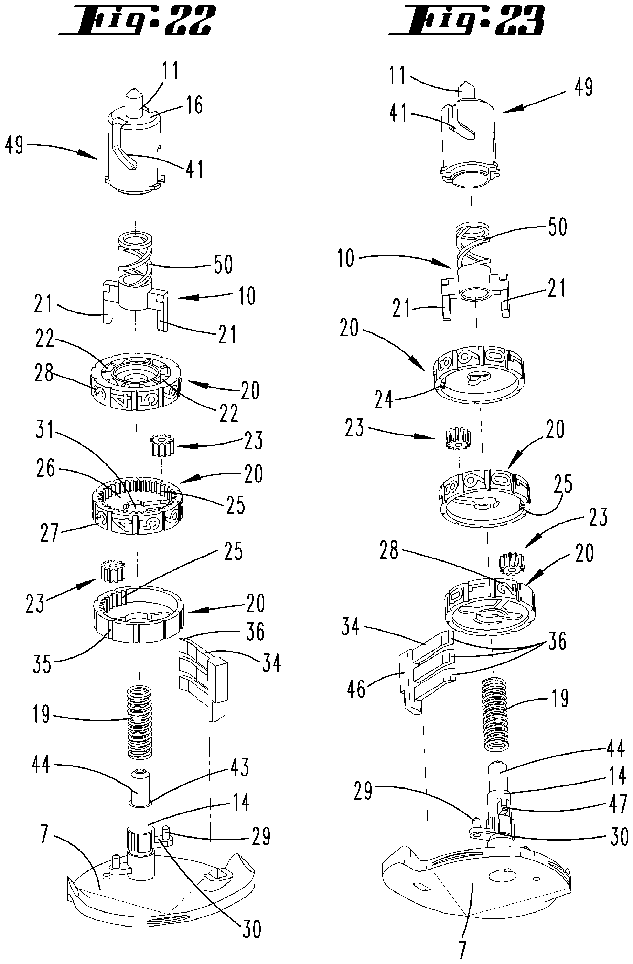

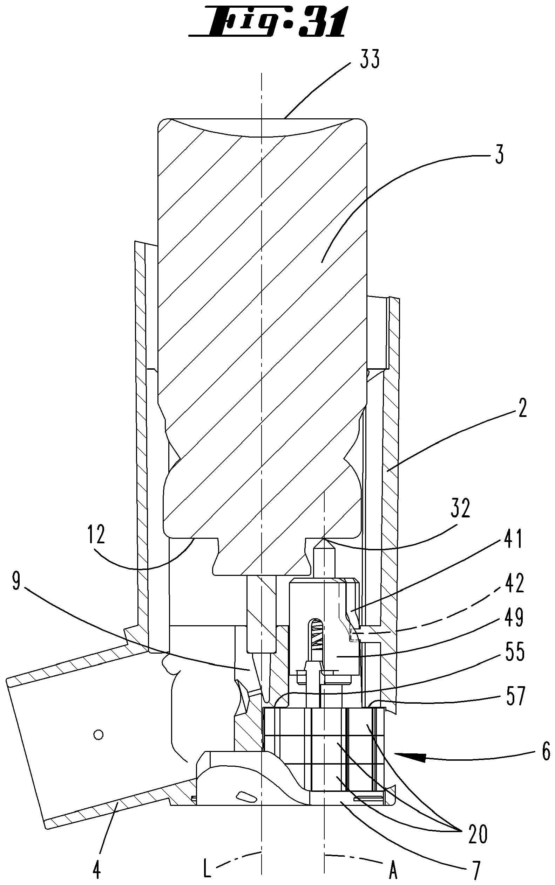

Aus den Explosionsdarstellungen der Figuren 22 und 23 ist erkennbar, dass im Unterschied zu der ersten Ausführungsform das Antriebsteil 10 nicht getriebemäßig, zur Erzeugung der erforderlichen Drehbewegung, mit der ersten Drehachse 14 zusammenwirkt. Hier wirkt vielmehr, und bevorzugt nur, das Beaufschlagungsteil 49 vermittels der außenseitig an dem Beaufschlagungsteil 49 ausgebildeten Kulisse 41 mit einem gehäusefesten Kulissenstein 42, siehe etwa Figur 31. Der Kulissenstein 42 ist bevorzugt unabhängig von der Bodenplatte 7 ausgebildet. Er ist bevorzugt wie beim Ausführungsbeispiel integraler, materialeinheitlicher, oder jedenfalls fest verbundener Teil des Gehäuses 2. Bevorzugt in dessen horizontal dem Ausgabefortsatz 8 bei eingesetztem Substanzbehältnis zugeordneten Bereich.

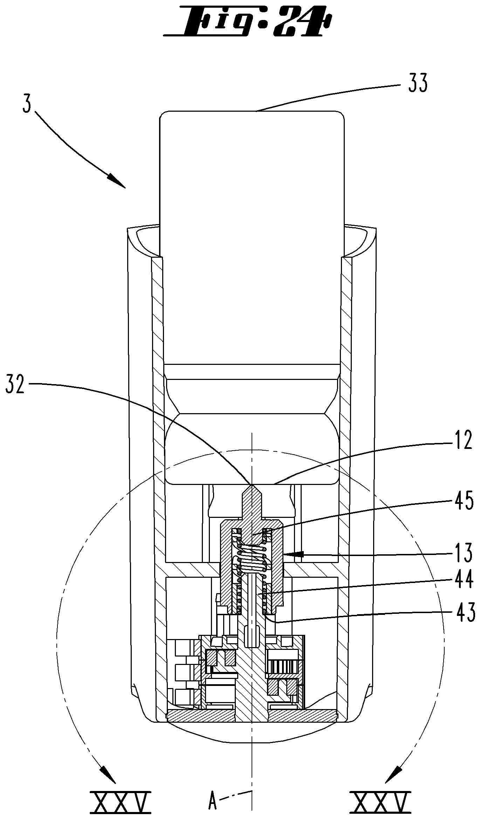

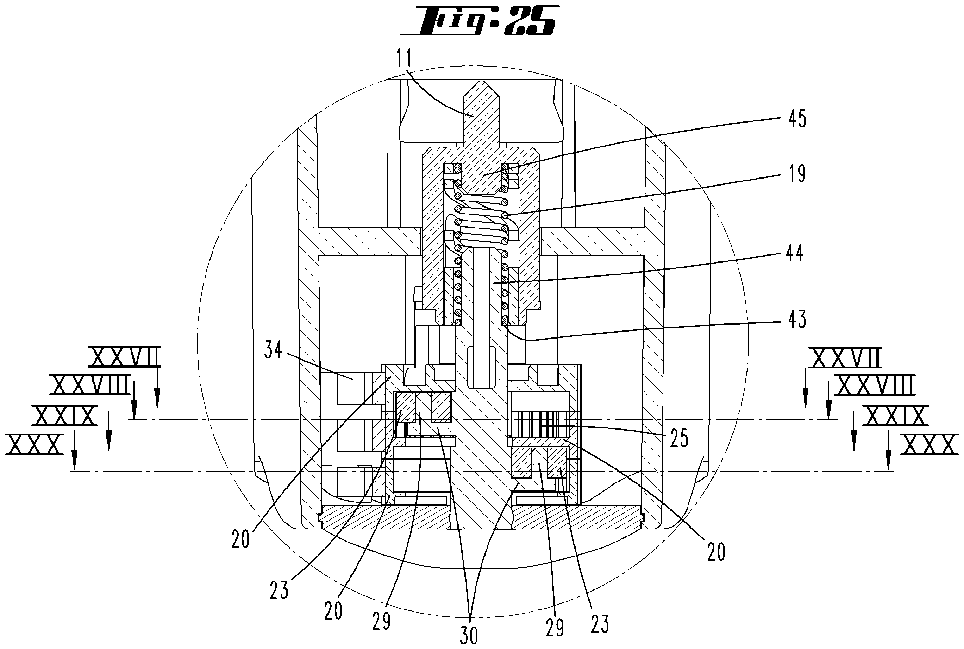

Die Feder 19 ist bevorzugt als Wendelfeder ausgebildet. Sie ist als solche auch bevorzugt koaxial zu der ersten Drehachse 24 angeordnet. Sie sitzt im Einzelnen, wie etwa Figur 24 zu entnehmen, auf einer Schulter der ersten Drehachse 14 auf. Ein Fortsetzungsabschnitt 44 der ersten Drehachse 14 erstreckt sich noch in Überdeckung zu der Feder 19. Andernendig sitzt die Feder 19, vergl. auch hierzu Figur 24, auf einem Halterungszapfen 45 des Antriebsteils 10, der sich im zusammengebauten Zustand, wie etwa auch Figur 25 zu entnehmen, koaxial zu der ersten Drehachse 14 erstreckt.

Das Antriebsteil 10 ist bei dieser Ausführungsform vertikal, d. h. in Richtung der ersten Drehachse relativ zu dem Beaufschlagungsteil 49 verfahrbar. Um die für die Drehbetätigung eines Zählrades erforderliche untere Stellung sicherzustellen, ist das Antriebsteil 10 mit einer weiteren Feder 50 zusammenwirkend vorgesehen. Beim Ausführungsbeispiel und bevorzugt stützt sich die Feder 50 innenseitig des Beaufschlagungsteils 49 ab. Auch ist wie dargestellt bevorzugt die Feder 50 materialeinheitlich, als Kunststofffeder, mit dem Antriebsteil 10 ausgebildet.

Dagegen ist die Feder 19 bevorzugt eine metallische Feder, insbesondere eine Stahlfeder.

Das Antriebsteil 10 ist in einem ersten Bereich, der bevorzugt innerhalb des Beaufschlagungsteils 49 angeordnet ist, hülsenartig gebildet. Das Antriebsteil 10 umfasst mit diesem hülsenartigen Bereich auch über einen Teil der axialen Erstreckung die Feder 19. Die Feder 19 ist in diesem Bereich also radial gesehen zwischen der ersten Drehachse 14 und dem Antriebsteil 10 angeordnet, siehe auch Figur 24.

Das Antriebsteil 10 setzt sich weiter, bevorzugt materialeinheitlich, in eine oder, wie bevorzugt beim Ausführungsbeispiel, zwei Eingriffsausformungen, die hier als Eingriffszapfen 21 ausgebildet sind, fort. Diese Eingriffsausformungen sind auch, bevorzugt und wie dargestellt, bezüglich einer Durchmesserlinie der ersten Drehachse oder des hülsenartigen Bereichs des Antriebsteils gegenüberliegend angeordnet.

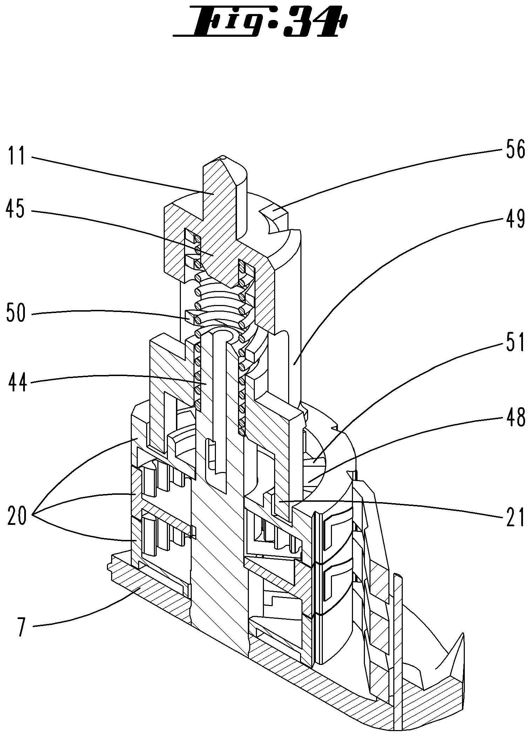

Eine Eingriffsausformung 21 greift jeweils in eine Eingriffsöffnung 22 des Zählrades ein. Hierbei liegt die Eingriffsausformung 21 in Drehrichtung des Zählrades an einer bevorzugt vertikalen, d. h. sich in Richtung der Drehachse erstreckenden Eingriffsfläche 48 an (siehe etwa Figur 34). Eine entgegen der Drehrichtung vorgesehene Gegenfläche 51 ist jedenfalls bei diesem zweiten Ausführungsbeispiel bevorzugt gekrümmt ausgebildet. Sie bildet eine Auflaufschräge für die Eingriffsausformung 21, die bei einem Rückhub des Beaufschlagungsteils entsprechend auch zurückgedreht wird und somit in eine nächste Eingriffsöffnung 22 einfährt. Abgeleitet aus der Rückdrehung und durch das Überlaufen der Auflaufschräge erfolgt die hierbei erforderliche Anhebung des Antriebsteils 10, das dann durch die Feder 50 wieder heruntergedrückt wird.

Insbesondere bei diesem Rückdrehen kommt es entsprechend zu einer relativen Bewegung in Richtung der Drehachse zwischen dem Beaufschlagungsteil und dem Eingriffsteil, so dass auch hierzu die genannte Feder 50 vorteilhaft ist.

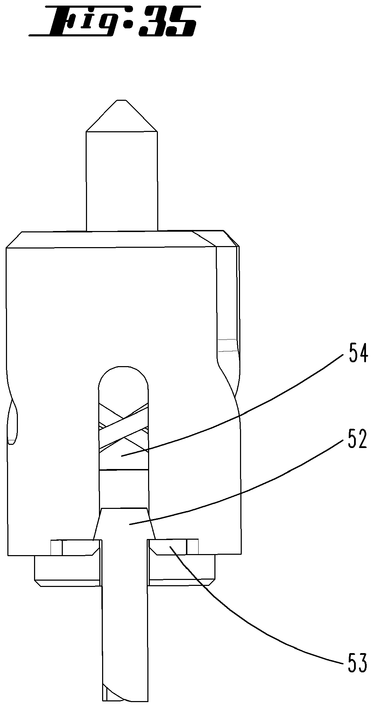

Das Antriebsteil 10 ist bevorzugt verliersicher an dem Beaufschlagungsteil 49 gehaltert. Wie sich aus Figur 35 ergibt, kann hierzu eine Rastverbindung vorgesehen sein. Eine Rastnase 52 an dem Eingriffsteil kann (bei der Montage) zum Überlaufen einer Gegenrast 53 an dem Beaufschlagungsteil ausgebildet sein. Zur gewünschten und erforderlichen axialen Relativbewegung zwischen dem Antriebsteil 10 und dem Beaufschlagungsteil 49 ist eine oder bevorzugt wie beim Ausführungsbeispiel zwei, zu dem noch weiter bevorzugt gegenüberliegende, Längsnuten 54 an dem Beaufschlagungsteil 49 ausgebildet.

Die Rückdrehsperre 34 weist drei fingerartige Sperrelemente mit jeweils einer Sperrnase 36 auf. Die Sperrelemente wurzeln in einem gemeinsamen, bevorzugt einteilig und materialeinheitlich mit den Sperrelementen ausgebildeten Halterungsabschnitt 46. Der Halterungsabschnitt 46 ist mit dem Bodenteil 7 fest verbunden, bevorzugt steckverbunden. Er ist relativ zu dem Bodenteil 7 nicht drehbar, so dass es zu einer elastischen Ausformung einer Rückdrehsperre 34 bei einer entsprechenden Drehung des zugeordneten Zählrades kommt.

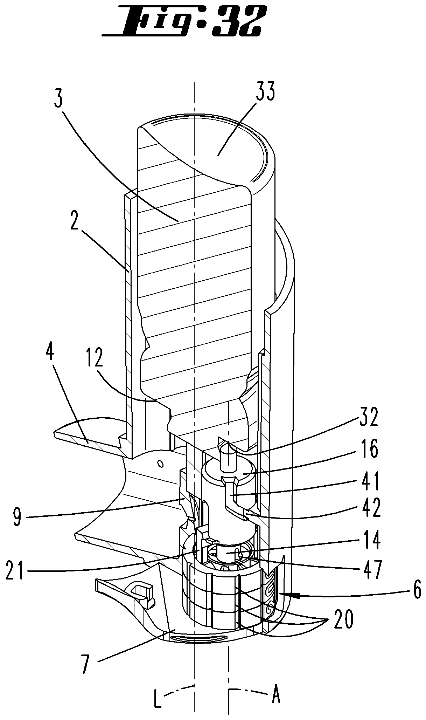

An der ersten Drehachse 14 ist weiter noch eine überlaufbare Rastnase 47 ausgebildet, so dass die Zählräder im zusammengesetzten Zustand axial gehalten sind, siehe etwa Figur 32.

Bei einer Benutzung des Handgerätes 1 der zweiten Ausführungsform ergibt sich Folgendes:

Der Nutzer drückt auf die obere Stirnseite 33 des Substanzbehältnisses 3, das hierdurch relativ zu dem Gehäuse 2 niedergedrückt wird. Dadurch wird das Beaufschlagungsteil 49 niedergedrückt, das zugleich über einen Teil seiner diesbezüglichen Bewegung in Richtung der Drehachse aufgrund der Zusammenwirkung zwischen der Getriebeausformung und dem Getriebeteil eine Bewegung um seine (vertikale) Längsachse durchführt.

Im Einzelnen besteht die Getriebeausformung aus einer Kulisse 41, wie sich beispielsweise aus Figur 32 ergibt, und das Getriebeteil aus einem in die Kulisse hineinragenden Kulissenstein 42 des Gehäuses 2.

Wie darüber hinaus ersichtlich ist die Kulisse 41 über einen Teil ihrer Länge so ausgebildet, dass es zu einer Drehung des Beaufschlagungsteils kommt. Über einen weiteren Teil verfährt das Beaufschlagungsteil 49 diesbezüglich ohne Drehung.

Mit dem Niederdrücken des Beaufschlagungsteils 49 wird die Feder 50, die auf das Antriebsteil 10 wirkt, komprimiert. Soweit noch nicht geschehen, wird das Antriebsteil 10 damit in seine bezüglich des Beaufschlagungsteils 49 gegebene untere Endstellung verlagert, so dass es zu einem sicheren Eingriff mit dem Zählrad kommt.

Durch das gleichzeitige Niederdrücken und Drehen des Beaufschlagungsteils erfolgt entsprechend zugleich eine solche Drehung des Antriebsteils 10 und damit des (obersten) Zählrades 20.

Nachdem von dem Benutzer das Substanzbehältnis 3 wieder freigegeben wird, bewegt sich dieses aufgrund einer in ihm selbst befindlichen Feder wieder in seine Ausgangsstellung zurück. Das Beaufschlagungsteil 49 folgt aufgrund der Vorspannung durch die Feder 19 unmittelbar dieser Rückstellbewegung. Hierbei kommt es entsprechend der getriebeartigen Zusammenwirkung des Beaufschlagungsteils mit dem Gehäuse 2 auch zu einer Rückdrehung des Beaufschlagungsteils 49.

Aufgrund der Drehkopplung zu dem Antriebsteil 10 bewegt sich dieses auch zugleich zurück. Infolge der Schrägflächen der Eingriffsöffnungen in dem Zahnrad 20 kann ein Eingriffsvorsprung des Antriebsteils 10 sich in eine vorgelagerte Eingriffsöffnung verlagern (ggf. können auch mehrere Eingriffsöffnungen überfahren werden).

Somit vollzieht nicht nur das Beaufschlagungsteil 49 sondern auch das Antriebsteil 10 bei einem Betätigungszyklus des Gerätes eine in Drehrichtung Hin- und Her-Bewegung.

Aufgrund der bei der Hin-Drehbewegung des Antriebsteils 10 erfolgenden Drehbewegung des Zählrades ist damit die Dosisausgabe bei dem Niederdrücken des Substanzbehältnisses gezählt. Die Rückdrehsperre verhindert ein Rückdrehen des Zählrades, während das Antriebsteil den zweiten Teil der Drehbewegung, die Her-Drehbewegung vollzieht. Es wird folglich jede Dosisabgabe gezählt. Es handelt sich um ein Einzeldosis-Zählwerk.

Im Hinblick auf beide Ausführungsformen ist der Figur 14 bzw. der Figur 31 zu entnehmen, dass die Aufnahme 9, konkret auf ihrer dem Stutzen 4 abgewandten Seite, eine Stufe 55 ausbildet, die den Aufnahmeraum im Hinblick auf die Aufnahme 9 für die Zählräder 20 schafft, so dass der Bauraum des Gehäuses 2 günstig ausgenutzt ist. Dies ist auch in den Querschnittsdarstellungen bspw. der Figuren 9, 8, 20 und 26 zu erkennen. Bei der zweiten Ausführungsform ist zusätzlich oder alternativ auch eine gehäuseseitige Stufe 57 vorgesehen, die grundsätzlich auch bei der ersten Ausführungsform alternativ oder zusätzlich vorgesehen sein kann.

Für den Benutzer, siehe etwa Figur 8, erscheint die Zahl der abgegebenen, oder der noch vorhandenen, wenn heruntergezählt wird, Dosen rückwärtig des Gehäuses, auf der dem Stutzen 4 abgewandten Seite. Die Zahl ist derart gegeben, dass sie in Längsrichtung des Gehäuses bzw. Erstreckungsrichtung des Substanzbehältnisses 3 abzulesen ist. Hierzu ist in dem Gehäuse eine Öffnung 58 vorgesehen. Durch die Öffnung 58, die bevorzugt langlochartig gebildet ist, sind die lesbaren Zeichen des einen Zählrads oder der mehreren Zählräder sichtbar. Die Öffnung kann mit einem transparenten Teil ausgefüllt sein. Sie kann aber auch als freie Öffnung gebildet sein, die also einen unmittelbaren Zugang zu dem einen oder mehreren Zählrad/-rädern insofern ermöglicht.

Hinsichtlich der Rückdrehsperre 34, die bevorzugt wie ersichtlich für jedes Zählwerk 20 einen eigenen Sperrfinger ausbildet, ist bevorzugt vorgesehen, dass diese mit einer Außenumfangsfläche eines Zählrades 20 zusammenwirkt, auf welcher auch die lesbaren Zeichen gegeben sind. In weiterer Einzelheit weist die Außenumfangsfläche hierzu in Richtung der ersten Drehachse sich erstreckende Rastkerbe 35 auf, in welche eine Sperrnase 36 der Rückdrehsperre 34 im Stillstand eingreift. Eine Rastkerbe 35, siehe etwa Figuren 9 bis 13 und 27 bis 30, ist mit einer flachen, überlaufbaren Flanke und einer steilen, im Wesentlichen an einer Durchmesserlinie in ihrem Verlauf (Querschnittsdarstellung) orientierten Flanke ausgebildet.

Letztere hindert das Rückdrehen, während erstere überlaufbar ist.

Die zweiten Zähne 25 sind bevorzugt immer über den vollen Umfang der entsprechenden Fläche ausgebildet. Sie können aber auch, wie etwa aus Figur 22 ersichtlich, nur über einen Teil der Fläche ausgebildet sein, etwa wenn für das Zählwerk tatsächlich nicht der volle mögliche Zifferbereich zur Anwendung kommt. Gleiches gilt hinsichtlich der lesbaren Zeichen, die bevorzugt eben Ziffern sind.

Das Zählwerk ist insbesondere auch günstig zu montieren. Ausgehend von dem Bodenteil 7 kann auf diesem die erste Drehachse und/oder Drehsperre 34 durch Steckmontage von oben befestigt werden. Die erste Drehachse 14 ist bevorzugt einteilig zugleich mit den zweiten Drehachsen 29 ausgebildet.

Auf die erste Drehachse 14 können sodann das eine oder die mehreren, beim den Ausführungsbeispielen drei, Zählräder aufgesteckt werden. Bei der zweiten Ausführungsform, was auch bei der ersten vorgesehen sein kann, sind sie durch die Rastnase 47 dann auch sogleich gehaltert.

Nach Montage eines unteren oder der zwei unteren Zählräder ist jeweils auch das Übertragungszahnrad in Steckmontage auf die zweite Drehachse 29 zu montieren.

Sodann kann, zweite Ausführungsform, die Feder 19 auf die erste Drehachse 14 aufgesteckt, weiter dann das Antriebsteil 10 und das Beaufschlagungsteil 49. Durch Niederdrücken des Beaufschlagungsteils 49 verrastet dieses mit dem Antriebsteil 10. Alternativ kann auch die Verrastung zuvor vorgenommen werden und die beiden Teile als Baugruppe aufgesetzt werden.

Bei der ersten Ausführungsform ist die Einheit von Antriebsteil 10 und Beaufschlagungsteil 49 gegeben, so dass hier nur ein Montagevorgang erforderlich ist.

Das so erstellte Zählwerk kann dann unten in das Gehäuse 2 eingesetzt werden. Dadurch, dass das Bodenteil 7 vorteilhafterweise größer gebildet ist als es für die ledigliche Montage des Zählwerks erforderlich ist, kann es auch günstig mit schon montiertem Zählwerk, insbesondere zum Einbau in das Gehäuse 2 gehandhabt werden. Hiermit ist auch eine vorteilhafte Ausrichtbarkeit gegeben. Das Beaufschlagungsteil 49, kombiniert mit dem Antriebsteil 10, kann bei der Vormontage so ausgerichtet werden, dass die - bevorzugt zweifach, gegenüberliegend - vorgesehenen Kulissen 41 zu den Kulissensteinen 42 ausgerichtet sind. Im Hinblick auf kleine Ausrichtungs-Ungenauigkeiten sind die Kulissen 41, siehe bspw. Fig. 34, bevorzugt mit einer sich in Umfangsrichtung des Beaufschlagungsteils 49 erweiternden Einführschräge 56 versehen.

Zur Durchführung einer Rastmontage des Bodenteils können, wie ersichtlich, an dem Bodenteil auch Rastnasen 56 ausgebildet sein.

Mit Ausnahme der Rückdrehsperre 34 sind alle Teile des Zählwerks mit der ersten Drehachse 14 verbunden oder hieran geführt.

Die vorstehenden Ausführungen dienen der Erläuterung der von der Anmeldung insgesamt umfassten Erfindungen, die den Stand der Technik durch die folgenden, der beanspruchten Erfindung ausdrücklich nicht gehörenden Merkmalskombinationen jeweils auch eigenständig weiterbilden, nämlich:
Ein Zählwerk, das dadurch gekennzeichnet ist, dass das Beaufschlagungsteil 49 drehbewegt ist und mit dem Antriebsteil 10 zur gemeinsamen Drehung verbunden ist.

Ein Zählwerk, das dadurch gekennzeichnet ist, dass das Beaufschlagungsteil 49 überlagert zu einer Bewegung in Richtung der Drehachse 14 drehbewegt ist und/oder, dass das Antriebsteil 10 und/oder das Beaufschlagungsteil 49 eine Getriebeausformung aufweist, wobei, bevorzugt, die Getriebeausformung zur Zusammenwirkung mit einem an der Drehachse 14 oder gesondert von der Drehachse 14 vorgesehenen Getriebeteil ausgebildet ist.

Ein Zählwerk, das dadurch gekennzeichnet ist, dass das Antriebsteil 10 koaxial zu der Drehachse 14 angeordnet ist und/ oder, dass das Antriebsteil 10 vor einer Verlagerung vollständig von dem Zählrad 20 getrennt ist und/oder, dass das Antriebsteil 10 auch vor einer Verlagerung an der (ersten) Drehachse 14 gefangen ist und/oder, dass die Getriebeausformungen aus einer Kulisse 18 an dem einen der Teile Antriebsteil 10 und (erste) Drehachse 14 und einem Kulissenstein 17 an dem anderen der Teile Antriebsteil 10 und (erste) Drehachse 14 bestehen und/oder, dass das Zählwerk 6 ein Bodenteil 7, ggf. als ein Teil eines Gehäuses des Handgerätes 1, umfasst.

Ein Zählwerk, das dadurch gekennzeichnet ist, dass eine das Antriebsteil 10 und/oder das Beaufschlagungsteil 9 beaufschlagende die Feder 19 sich auf einer Innenseite des Bodenteils 7 abstützt, wobei, bevorzugt, die erste Drehachse 14 fest mit dem Bodenteil 7 verbunden ist und/oder, dass mehrere Zählräder 20 vorgesehen sind, wobei, bevorzugt, alle Zählräder 20 koaxial angeordnet sind und/oder, dass das Antriebsteil 10 als Eingriffsausformung einen Eingriffszapfen 21 aufweist, wobei, weiter bevorzugt, eine Eingriffsausformung achsparallel zu der Drehachse 14 verläuft.

Ein Zählwerk, das dadurch gekennzeichnet ist, dass ein unmittelbar dem Antriebsteil 10 zugeordnetes Zählrad 20 eine Eingriffsöffnung 22 aufweist, wobei, bevorzugt, die Eingriffsöffnung 22 zu einer Aufnahme einer Eingriffsausnehmung ausgebildet und angeordnet ist und/oder, dass zwischen zwei in Richtung der Drehachse 14 unmittelbar benachbarten Zählrädern 20 ein Übertragungszahnrad 23 angeordnet ist, das sich quer zu seiner (zweiten) Drehachse 29 in Überdeckung zu beiden Zählrädern 20 erstreckt.

Ein Zählwerk, das dadurch gekennzeichnet ist, dass ein Übertragungszahnrad 22 drehbar an einem Ausleger 30 der ersten Drehachse 14 gehaltert ist, wobei, bevorzugt, zwei Übertragungszahnräder 23 vorgesehen sind und/oder zwei Ausleger 30 vorgesehen sind, wobei, bevorzugt, die Ausleger 30 in Richtung der ersten Drehachse 14 beabstandet sind.

Ein Zählwerk, das dadurch gekennzeichnet ist, dass eine die Federkraft für das Antriebsteil 10 und/oder das Beaufschlagungsteil 49 erbringende Feder 19 sich seitlich zu einem Zählrad 20 und in Richtung der ersten Drehachse 14 das Zählrad 20 übergreifend erstreckt und/oder eines oder mehrere der Zählräder 20 mit einer Rückdrehsperre 34 zusammenwirken, wobei, bevorzugt, eine Rückdrehsperre 34 als ein auf eine die Zeichen aufweisende Außen-Umfangsfläche eines Zählrades 20 einwirkendes Sperrteil ausgebildet ist, wobei, weiter bevorzugt, eine Bewegungsebene des Sperrteils sich quer zu einer Erstreckung der ersten Drehachse 14 erstreckt und/oder das Sperrteil an dem Bodenteil 7 befestigt ist.

Ein Handgerät, das dadurch gekennzeichnet ist, dass das Zählwerk 6 ein Bodenteil 7 aufweist, das einen Teil des Gehäuses 2 bilden kann und in dieses einsetzbar sein kann, wobei sich das Bodenteil 7 in Richtung der Längsachse L des Substanzbehältnisses 3 zumindest teilweise in Überdeckung zu diesem befindet, und dass das Bodenteil 7 eine feststehende (erste) Drehachse 14 für das Zählrad 6 trägt, auf welcher bevorzugt auch das Antriebsteil 10 geführt ist, wobei sich das Antriebsteil 10 bzgl. des Zählrades 20 auf der Seite des Substanzbehältnisses 3 befindet.

Ein Handgerät, das dadurch gekennzeichnet ist, dass das Antriebsteil 10 und/oder das Beaufschlagungsteil 9 eine Getriebeausformung aufweist zur Zusammenwirkung mit einem gehäusefesten Getriebeteil, zur Erzeugung einer Drehbewegung des Antriebsteils 10 relativ zu dem Gehäuse 2 im Zuge des Ausgabehubes.

**Bezugszeichenliste**

| | | | |
|---|---|---|---|
| | | 28 | Zeichen |
| 1 | Handgerät | 29 | zweite Drehachse |
| 2 | Gehäuse | 30 | Ausleger |
| 3 | Substanzbehältnis | 31 | Öffnung |
| 4 | Ausgabestutzen | 32 | Berührungsspitze |
| 5 | Verschlusskappe | 33 | obere Stirnseite |
| 6 | Zählwerk | 34 | Rückdrehsperre |
| 7 | Bodenteil | 35 | Rastkerbe |
| 8 | Ausgabefortsatz | 36 | Sperrnase |
| 9 | Aufnahme | 37 | Einführabschnitt |
| 10 | Antriebsteil | 38 | Drehabschnitt |
| 11 | Beaufschlagungsfortsatz | 39 | Kulissenfläche |
| 12 | Stirnfläche | 40 | Nut |
| 13 | Führungsabschnitt | 41 | Kulisse |
| 14 | erste Drehachse | 42 | Kulissenstein |
| 15 | Stirnende | 43 | Schulter |
| 16 | Stirnfläche | 44 | Fortsetzungsabschnitt |
| 17 | Kulissenstein | 45 | Halterungszapfen |
| 18 | Kulisse | 46 | Halterungsabschnitt |
| 19 | Feder | 47 | Rastnase |
| 20 | Zählrad | 48 | Eingriffsfläche |
| 21 | Eingriffszapfen | 49 | Beaufschlagungsteil |
| 22 | Eingriffsöffnung | 50 | Feder |
| 23 | Übertragungszahnrad | 51 | Gegenfläche |
| 24 | erste Zähne | 52 | Rastnase |
| 25 | zweite Zähne | 53 | Gegenrast |
| 26 | Boden | 54 | Längsnut |
| 27 | Umfangs-Mantelteil | 55 | Stufe |
| | | | |
| 56 | Einführschräge | | |
| 57 | Stufe | | |
| 58 | Öffnung | | |
| | | | |
| A | geometrische Achse | | |
| L | Längsachse | | |

## Patentansprüche

1. Zählwerk (6) für ein Handgerät (1) zur Ausgabe einer pharmazeutischen Substanz, insbesondere eines Inhaliermedikamentes, mit mindestens einem, lesbare Zeichen aufweisenden Zählrad (20), wobei das Zählrad (20) um eine Drehachse (14) drehbar ist und zur Dreheinwirkung auf das Zählrad (20) ein Antriebsteil (10) für das Zählrad (20) vorgesehen ist, welches Antriebsteil(10) eine Eingriffsausformung zur Zusammenwirkung mit dem Zählrad (20) aufweist, wobei weiter ein gleichfalls in der Erstreckungsrichtung der Drehachse (14) gegen Federkraft relativ zu dem Zählrad (20) verlagerbares Beaufschlagungsteil (49) vorgesehen ist, wobei das Beaufschlagungsteil (49) drehbar ist und mit dem Antriebsteil (10) zur gemeinsamen Drehung verbunden ist, dadurch gekenzeichnet, dass das Antriebsteil (10) einen Führungsabschnitt (13) aufweist, mit dem es im zusammengesetzten Zustand des Zählwerks (6) auf der als feststehender Zapfen ausgebildeten ersten Drehachse (14) des Zählwerks aufsitzt.

2. Zählwerk nach Anspruch 1, **dadurch gekennzeichnet, dass** das Beaufschlagungsteil (49) überlagert zu einer Bewegung in Richtung der Drehachse (14) drehbewegt ist und/ oder, bevorzugt, dass das Antriebsteil (10) und/oder das Beaufschlagungsteil (49) eine Getriebeausformung aufweist, wobei, bevorzugt, die Getriebeausformung zur Zusammenwirkung mit einem an der Drehachse (14) oder gesondert von der Drehachse (14) vorgesehenen Getriebeteil ausgebildet ist.

3. Zählwerk nach einem der vorhergehenden Ansprüche, **dadurch gekennzeichnet, dass** das Antriebsteil (10) koaxial zu der Drehachse (14) angeordnet ist und/oder dass das Antriebsteil (10) vor einer Verlagerung vollständig von dem Zählrad (20) getrennt ist und/oder dass das Antriebsteil (10) auch vor einer Verlagerung an der ersten Drehachse (14) gefangen ist.

4. Zählwerk nach einem der Ansprüche 2 oder 3, **dadurch gekennzeichnet, dass** die Getriebeausformungen aus einer Kulisse (18) an dem einen der Teile Antriebsteil (10) und erste Drehachse (14) und einem Kulissenstein (17) an dem anderen der Teile Antriebsteil (10) und erste Drehachse (14) bestehen.

5. Zählwerk nach einem der vorhergehenden Ansprüche, **dadurch gekennzeichnet, dass** das Zählwerk (6) ein Bodenteil (7), ggf. als ein Teil eines Gehäuses des Handgerätes (1), umfasst und/oder dass eine das Antriebsteil (10) und/oder das Beaufschlagungsteil (9) beaufschlagende Feder (19) sich auf einer Innenseite des Bodenteils (7) abstützt.

6. Zählwerk nach einem der vorhergehenden Ansprüche, **dadurch gekennzeichnet, dass** die erste Drehachse (14) fest mit dem Bodenteil (7) verbunden ist.

7. Zählwerk nach einem der vorhergehenden Ansprüche, **dadurch gekennzeichnet, dass** eine die Federkraft für das Antriebsteil (10) und/oder das Beaufschlagungsteil (49) erbringende Feder (19) sich seitlich zu dem Zählrad (20) und in Richtung der ersten Drehachse (14) das Zählrad (20) übergreifend erstreckt.

8. Zählwerk nach einem der vorhergehenden Ansprüche, **dadurch gekennzeichnet, dass** mehrere Zählräder (20) vorgesehen sind, wobei, bevorzugt, alle Zählräder (20) koaxial angeordnet sind.

9. Zählwerk nach einem der vorhergehenden Ansprüche, **dadurch gekennzeichnet, dass** eines oder mehrere der Zählräder (20) mit einer Rückdrehsperre (34) zusammenwirken.

10. Zählwerk nach Anspruch 9, **dadurch gekennzeichnet, dass** die Rückdrehsperre (34) als ein auf eine die Zeichen aufweisende Außen-Umfangsfläche eines Zählrades (20) einwirkendes Sperrteil ausgebildet ist.

11. Zählwerk nach Anspruch 10, **dadurch gekennzeichnet, dass** eine Bewegungsebene des Sperrteils sich quer zu einer Erstreckung der ersten Drehachse (14) erstreckt und/oder das Sperrteil an dem Bodenteil (7) befestigt ist.

12. Zählwerk nach einem der vorhergehenden Ansprüche, **dadurch gekennzeichnet, dass** das Antriebsteil (10) als Eingriffsausformung einen Eingriffszapfen (21) aufweist und/oder dass die Eingriffsausformung achsparallel zu der Drehachse (14) verläuft und/oder dass das Zählrad (20), gegebenenfalls ein unmittelbar dem Antriebsteil (10) zugeordnetes Zählrad (20), eine Eingriffsöffnung (22) aufweist, wobei, bevorzugt die Eingriffsöffnung (22) zu einer Aufnahme einer Eingriffsausnehmung ausgebildet und angeordnet ist.

13. Zählwerk nach einem der Ansprüche 8 bis 12, **dadurch gekennzeichnet, dass** zwischen zwei in Richtung der Drehachse (14) unmittelbar benachbarten Zählrädern (20) ein Übertragungszahnrad (23) angeordnet ist, das sich quer zu seiner zweiten Drehachse (29) in Überdeckung zu beiden Zählrädern (20) erstreckt, wobei, bevorzugt, ein Übertragungszahnrad (22) drehbar an einem Ausleger (30) der ersten Drehachse (14) gehaltert ist und/oder zwei Übertragungszahnräder (23) vorgesehen sind und/oder zwei Ausleger (30) vorgesehen sind, wobei weiter bevorzugt die Ausleger (30) in Richtung der ersten Drehachse (14) beabstandet sind.

14. Handgerät (1) zur Ausgabe sprühfähiger Substanzen, insbesondere Inhaliermedikamenten, mit einem Gehäuse (2), wobei in dem Gehäuse (2) ein mit einem Ausgabehub zu betätigendes Substanzbehältnis (3), das eine Längsachse (L) aufweist, und ein Zählwerk (6) aufgenommen sind, wobei das Zählwerk (6) mindestens ein, lesbare Zeichen aufweisendes Zählrad (20) und ein zum Drehen des Zählrades (20) auf dieses einwirkendes, bei dem Ausgabehub von dem Substanzbehältnis (3) verlagertes Antriebsteil (10) aufweist, **dadurch gekennzeichnet, dass** das Zählwerk (6) ein Bodenteil (7) aufweist, das einen Teil des Gehäuses (2) bildet und in dieses eingesetzt ist, wobei sich das Bodenteil (7) in Richtung der Längsachse (L) des Substanzbehältnisses (3) zumindest teilweise in Überdeckung zu diesem befindet, und dass das Bodenteil (7) eine feststehende Drehachse (14) für das Zählrad (6) trägt, wobei sich das Antriebsteil (10) bzgl. des Zählrades (20) auf der Seite des Substanzbehältnisses (3) befindet.

15. Handgerät nach Anspruch 14, **dadurch gekennzeichnet, dass** das Antriebsteil (10) und/oder das Beaufschlagungsteil (9) eine Getriebeausformung aufweist zur Zusammenwirkung mit einem gehäusefesten Getriebeteil, zur Erzeugung einer Drehbewegung des Antriebsteils (10) relativ zu dem Gehäuse (2) im Zuge des Ausgabehubes.

## Claims

1. A counter (6) for a handheld device (1) for dispensing pharmaceutical substances, particularly an inhalation medication, with at least one counter wheel (20) that features legible characters, wherein the counter wheel (20) is rotatable about a rotational axis (14) and a drive part (10) for the counter wheel (20) is provided in order to rotationally act upon the counter wheel (20), wherein said drive part (10) features a shaped engagement section for cooperating with the counter wheel (20), and wherein an acting part (49) is furthermore provided and can likewise be displaced relative counter wheel (20) in the direction of the rotational axis (14) against a spring force, wherein the acting part (49) is rotationally moveable and connected to the drive part (10) in order to rotate jointly therewith, **characterized in that** the drive part (10) features a guide section (13), by means of which it is seated on the first rotational axis (14) of the counter realized in the form of a stationary pin in the assembled state of the counter (6).

2. The counter according to claim 1, **characterized in that** the rotational motion of the acting part (49) is superimposed with a motion in the direction of the rotational axis (14) and / or, preferably, that the drive part (10) and/or the acting part (49) feature a shaped gear section, wherein, preferably the shaped gear section is designed for cooperating with a gear part provided on the rotational axis (14) or separately of the rotational axis (14).

3. The counter according to one of the preceding claims, **characterized in that** the drive part (10) is arranged coaxial to the rotational axis (14) and/ or that the drive part (10) is completely separated from the counter wheel (20) prior to a displacement and/ or that the drive part (10) is also captured on the first rotational axis (14) prior to a displacement.

4. The counter according to one of claims 2 or 3, **characterized in that** the shaped gear sections consist of a slotted link (18) on one of the components drive part (10) and first rotational axis (14), as well as a slide block (17) on the other of the components drive part (10) and first rotational axis (14).

5. The counter according to one of the preceding claims, **characterized in that** the counter (6) comprises a bottom part (7) that, if applicable, forms part of a housing of the handheld device (1) and/ or that a spring (19) acting upon the drive part (10) and/or the acting part (9) is supported on an inner side of the bottom part (7).

6. The counter according to one of the preceding claims, **characterized in that** the first rotational axis (14) is rigidly connected to the bottom part (7).

7. The counter according to one of the preceding claims, **characterized in that** a spring (19), which generates the spring force for the drive part (10) and/ or the acting part (49), extends laterally of a counter wheel (20) and in the direction of the first rotational axis (14) such that it overlaps the counter wheel (20).

8. The counter according to one of the preceding claims, **characterized in that** several counter wheels (20) are provided, wherein preferably all counter wheels (20) are arranged coaxial.

9. The counter according to one of the preceding claims, **characterized in that** one or more of the counter wheels (20) cooperate with a reverse lock (34).

10. The counter according to claim 9, **characterized in that** a reverse lock (34) is realized in the form of a locking part that acts upon an outer circumferential surface of a counter wheel (20) featuring the characters.

11. The counter according to claim 10, **characterized in that** a motion plane of the locking part extends transverse to the first rotational axis (14) and/or the locking part is fixed on the bottom part (7).

12. The counter according to one of the preceding claims, **characterized in that** the drive part (10) features a shaped engagement section in the form of an engagement pin (21) and/ or that a shaped engagement section extends axially parallel to the rotational axis (14) and/ or that a counter wheel (20), if applicable a counter wheel (20) assigned directly to the drive part (10), features an engagement opening (22), wherein, preferably, the engagement opening (22) is designed and arranged for accommodating a shaped engagement section.

13. The counter according to one of the claims 8 to 12, **characterized in that** a transmission gearwheel (23) is provided between two counter wheels (20) that are arranged directly adjacent to one another in the direction of the rotational axis (14), said transmission gearwheel extends transverse to its second rotational axis (29) such that it overlaps both counter wheels (20), wherein, preferably, a transmission gearwheel (22) is rotatably held on a radial arm (30) of the first rotational axis (14) and/or two transmission gearwheels (23) are provided and/or two radial arms (30) are provided, wherein further preferably the radial arms (30) are spaced apart in the direction of the first rotational axis (14).

14. A handheld device (1) for dispensing sprayable substances, particularly an inhalation medication, with a housing (2), wherein a substance container (3) with a longitudinal axis (L), which is actuated by means of a dispensing stroke, and a counter (6) are accommodated in the housing (2), and wherein the counter (6) comprises at least one counter wheel (20) that features legible characters, as well as a drive part (10) that rotationally acts upon the counter wheel (20) and is displaced during a dispensing stroke of the substance container (3), **characterized in that** the counter (6) features a bottom part (7), which forms part of the housing (2) and is inserted therein, wherein the bottom part (7) at least partially overlaps the substance container (3) in the direction of the longitudinal axis (L) thereof, and that the bottom part (7) carries a stationary rotational axis (14) for the counter wheel (6), wherein the drive part (10) is located on the side of the substance container (3) referred to the counter wheel (20).

15. The handheld device according to claim 14, **characterized in that** the drive part (10) and/or the acting part (9) feature a shaped gear section for cooperating with a stationary gear part of the housing in order to realize a rotational motion of the drive part (10) relative to the housing (2) during the course of the dispensing stroke.

## Revendications

1. Compteur (6) pour un dispositif portatif (1) pour la distribution d'une substance pharmaceutique, en particulier un médicament à inhaler, avec au moins une roue de comptage (20) présentant des caractères lisibles, dans lequel la roue de comptage (20) peut tourner autour d'un axe de rotation (14), et un élément d'entraînement (10) est prévu pour la roue de comptage (20) pour faire tourner la roue de comptage (20), lequel élément d'entraînement (10) comprend une formation d'engagement destinée à coopérer avec la roue de comptage (20), dans lequel est prévu en outre un élément d'actionnement (49) également déplaçable par rapport à la roue de comptage (20) dans la direction d'extension de l'axe de rotation (14) à l'encontre d'une force élastique, dans lequel l'élément d'actionnement (49) peut tourner et est relié à l'élément d'entraînement (10) pour une rotation commune, **caractérisé en ce que** l'élément d'entraînement (10) comprend une partie de guidage (13) avec laquelle, dans l'état assemblé du compteur (6), il est placé sur le premier axe de rotation (14) du compteur réalisé sous la forme d'une broche fixe.

2. Compteur selon la revendication 1, **caractérisé en ce que** l'élément d'actionnement (49) est déplaçable en rotation en superposition avec un mouvement dans la direction de l'axe de rotation (14) et/ou, de préférence, **en ce que** l'élément d'entraînement (10) et/ou l'élément d'actionnement (49) comprend une formation de transmission, dans lequel, de préférence, la formation de transmission est formée pour coopérer avec un élément de transmission prévu sur l'axe de rotation (14) ou séparément de l'axe de rotation (14) .

3. Compteur selon l'une quelconque des revendications précédentes, **caractérisé en ce que** l'élément d'entraînement (10) est agencé coaxialement par rapport à l'axe de rotation (14) et/ou **en ce que** l'élément d'entraînement (10) est complètement séparé de la roue de comptage (20) avant un déplacement et/ou **en ce que** l'élément d'entraînement (10) est également captif du premier axe de rotation (14) avant même un déplacement.

4. Compteur selon l'une des revendications 2 ou 3, **caractérisé en ce que** les formations de transmission sont constituées d'une coulisse (18) sur l'une des pièces parmi l'élément d'entraînement (10) et le premier axe de rotation (14), et d'un coulisseau (17) sur l'autre des pièces parmi l'élément d'entraînement (10) et le premier axe de rotation (14).

5. Compteur selon l'une des revendications précédentes, **caractérisé en ce que** le compteur (6) comporte une partie inférieure (7), le cas échéant sous la forme d'une partie du boîtier du dispositif portatif (1), et/ou **en ce que** l'élément d'entraînement (10) et/ou **en ce qu'**un ressort (19) agissant sur l'élément d'entraînement (10) et/ou l'élément d'actionnement (9) est en appui sur un côté intérieur de la partie inférieure (7).

6. Compteur selon l'une des revendications précédentes, **caractérisé en ce que** le premier axe de rotation (14) est lié fixement à la partie inférieure (7).

7. Compteur selon l'une des revendications précédentes, **caractérisé en ce qu'**un ressort (19) fournissant la force élastique pour l'élément d'entraînement (10) et/ou l'élément d'actionnement (49) s'étend latéralement par rapport à la roue de comptage (20) et dans la direction du premier axe de rotation (14) de la roue de comptage (20) en recouvrement de la roue de comptage (20).

8. Compteur selon l'une des revendications précédentes, **caractérisé en ce que** plusieurs roues de comptage (20) sont prévues, dans lequel, de préférence, toutes les roues de comptage (20) sont agencées coaxialement.

9. Compteur selon l'une des revendications précédentes, **caractérisé en ce qu'**une ou plusieurs des roues de comptage (20) coopèrent avec un verrou d'arrêt de la rotation en arrière (34).

10. Compteur selon la revendication 9, **caractérisé en ce que** le verrou d'arrêt de la rotation en arrière (34) est réalisé sous la forme d'une pièce d'arrêt agissant sur une surface périphérique externe d'une roue de comptage (20) présentant les caractères.

11. Compteur selon la revendication 10, **caractérisé en ce qu'**un plan de déplacement de la pièce d'arrêt s'étend transversalement à une extension du premier axe de rotation (14) et/ou **en ce que** la pièce d'arrêt est fixée à la partie inférieure (7).

12. Compteur selon l'une des revendications précédentes, **caractérisé en ce que** l'élément d'entraînement (10) comprend une broche d'engagement (21) en tant que formation d'engagement et/ou **en ce que** la formation d'engagement s'étend axialement parallèle à l'axe de rotation (14) et/ou **en ce que** la roue de comptage (20), le cas échéant une roue de comptage (20) associée directement à l'élément d'entraînement (10), comprend une ouverture d'engagement (22), dans lequel, de préférence, l'ouverture d'engagement (22) est formée et agencée pour recevoir une cavité d'engagement.

13. Compteur selon l'une des revendications 8 à 12, **caractérisé en ce qu'**entre deux roues de comptage (20) directement adjacentes dans la direction de l'axe de rotation (14) est agencée une roue dentée de transmission (23) qui s'étend transversalement à son deuxième axe de rotation (29) en recouvrement avec les deux roues de comptage (20), dans lequel, de préférence, une roue dentée de transmission (22) est fixée de manière rotative sur un bras (30) du premier axe de rotation (14) et/ou deux roues dentées de transmission (23) sont prévues et/ou deux bras (30) sont prévus, dans lequel, en outre, de préférence, les bras (30) sont espacés dans la direction du premier axe de rotation (14).

14. Dispositif portatif (1) pour distribuer des substances pulvérisables, en particulier des médicaments à inhaler, comprenant un boîtier (2), dans lequel un conteneur de substance (3) à actionner avec une course de distribution, qui présente un axe longitudinal (L), et un compteur (6) sont reçus dans le boîtier (2), dans lequel le compteur (6) comprend au moins une roue de comptage (20) présentant des caractères lisibles et un élément d'entraînement (10) agissant sur la roue de comptage (20) pour la faire tourner et qui est déplacé pendant une course de distribution du conteneur de substance (3), **caractérisé en ce que** le compteur (6) comprend une partie inférieure (7) qui forme une partie du boîtier (2) et qui y est insérée, dans lequel la partie inférieure (7) dans la direction de l'axe longitudinal (L) du conteneur de substance (3) est au moins partiellement en recouvrement avec celui-ci, et **en ce que** la partie inférieure (7) est au moins partiellement en recouvrement avec conteneur de substance (3) dans la direction de son axe longitudinal (L), et **en ce que** la partie inférieure (7) porte un axe de rotation fixe (14) pour le compteur (6), dans lequel l'élément d'entraînement (10) est situé sur le côté du conteneur de substance (3) par rapport à la roue de comptage (20) .

15. Dispositif portatif selon la revendication 14, **caractérisé en ce que** l'élément d'entraînement (10) et/ou l'élément d'actionnement (9) comprend une formation de transmission pour coopérer avec un élément de transmission fixe du boîtier pour générer un mouvement de rotation de l'élément d'entraînement (10) par rapport au boîtier (2) lors de la course de distribution.
